(19) Europäisches Patentamt
European Patent Office
Office européen des brevets

(11) **EP 3 887 810 B1**

(12) **EUROPÄISCHE PATENTSCHRIFT**

(45) Veröffentlichungstag und Bekanntmachung des Hinweises auf die Patenterteilung:
**10.09.2025   Patentblatt 2025/37**

(21) Anmeldenummer: **19813306.8**

(22) Anmeldetag: **29.11.2019**

(51) Internationale Patentklassifikation (IPC):
**G01N 27/02** (2006.01)

(52) Gemeinsame Patentklassifikation (CPC):
**G01N 27/026; G01R 27/22;** G01N 27/028;
G01N 33/48735; G01R 1/06

(86) Internationale Anmeldenummer:
**PCT/EP2019/083114**

(87) Internationale Veröffentlichungsnummer:
**WO 2020/109565 (04.06.2020 Gazette 2020/23)**

(54) **VERFAHREN UND SENSOR ZUM BESTIMMEN EINES DIE IMPEDANZ EINER SUSPENSION ANZEIGENDEN WERTS**

METHOD AND SENSOR FOR DETERMINING A VALUE INDICATIVE OF THE IMPEDANCE OF A SUSPENSION

METHODE ET CAPTEUR POUR DETERMINER UNE VALEUR INDICATIVE DE L'IMPEDENCE D'UNE SUSPENSION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.11.2018   DE 102018130487**

(43) Veröffentlichungstag der Anmeldung:
**06.10.2021   Patentblatt 2021/40**

(73) Patentinhaber: **Hamilton Bonaduz AG
7402 Bonaduz (CH)**

(72) Erfinder:
• **VERSCHININ, Valentin
7000 Chur (CH)**
• **IMHOF, Manuel
7012 Felsberg (CH)**

(74) Vertreter: **Schmitt-Nilson Schraud Waibel Wohlfrom
Patentanwälte Partnerschaft mbB
Pelkovenstraße 143
80992 München (DE)**

(56) Entgegenhaltungen:
**US-A1- 2017 071 552     US-A1- 2017 328 880**

• **RAHMAN A R A ET AL:** "Cell culture monitoring by impedance mapping using a multielectrode scanning impedance spectroscopy system (CellMap); Multielectrode scanning impedance spectroscopy", PHYSIOLOGICAL MEASUREMENT, INSTITUTE OF PHYSICS PUBLISHING, BRISTOL, GB, vol. 29, no. 6, 1 June 2008 (2008-06-01), pages S227 - S239, XP020137132, ISSN: 0967-3334
• **GUOFENG QIAO ET AL:** "Bioimpedance Analysis for the Characterization of Breast Cancer Cells in Suspension", IEEE TRANSACTIONS ON BIOMEDICAL ENGINEERING, IEEE SERVICE CENTER, PISCATAWAY, NJ, USA, vol. 59, no. 8, 1 August 2012 (2012-08-01), pages 2321 - 2329, XP011490168, ISSN: 0018-9294, DOI: 10.1109/ TBME.2012.2202904
• **HARRIS C M ET AL:** "Dielectric permittivity of microbial suspensions at radio frequencies: a novel method for the real-time estimation of microbial biomass", ENZYME AND MICROBIAL TECHNOLOGY, STONEHAM, MA, US, vol. 9, no. 3, 1 March 1987 (1987-03-01), pages 181 - 186, XP023689236, ISSN: 0141-0229, [retrieved on 19870301], DOI: 10.1016/0141-0229(87)90075-5

EP 3 887 810 B1

**Beschreibung**

Die vorliegende Erfindung liegt auf dem Gebiet der Impedanzspektroskopie von Suspensionen, insbesondere Zellsuspensionen. Insbesondere betrifft die vorliegende Erfindung das Bestimmen der Impedanz einer Suspension bzw. das Bestimmen eines die Impedanz einer Suspension anzeigenden Werts. Weiter insbesondere betrifft die vorliegende Erfindung ein Verfahren und einen Sensor zum Bestimmen eines die Impedanz einer Suspension anzeigenden Werts.

Elektrische Impedanzspektroskopieverfahren werden als Messverfahren zur zerstörungsfreien in-situ und in-vivo Bestimmung von frequenzabhängigen passiven elektrischen Eigenschaften von Suspensionen verwendet. Der Begriff Suspension bezeichnet dabei die Verteilung kleiner Teilchen eines Stoffs oder Stoffgemischs in einer Flüssigkeit. Ein Beispiel einer Suspension, die mit elektrischen Impedanzspektroskopieverfahren analysiert wird, ist eine Substanz aus einer Flüssigkeit und darin aufgenommenen biologischen Zellen, in ihrer Gesamtheit hierin als Zellpopulation bezeichnet. Die oben genannten frequenzabhängigen passiv-elektrischen Eigenschaften der Zellpopulation können unter anderem Auskunft über die Anzahl der lebenden Zellen und/oder Größe der Zellen und/oder Homogenität der Zellen geben.

Rahman A R A et al, "Cell culture monitoring by impedance mapping using a multielectrode scanning impedance spectroscopy system (CellMap)", Physiological Measurement, Institute of physics publishing, Bristiol, GB, Band 29, Nr. 6, 1. Juni 2008, offenbart ein Impedanzsensorsystem, das die Zellverteilung in einem Zellkulturraum durch Aufnehmen der Impedanz an verschiedenen Stellen abbilden kann. Dabei kommt eine radiale Elektrodenanordnung zum Einsatz. Eine multidimensionale Charkterisierung der Zellkulturen wird ermöglicht.

Bisherige Sensoren und Impedanzspektroskopieverfahren sind bezüglich der Genauigkeit der Messergebnisse nicht immer vollkommen zufriedenstellend. Auch kann die Qualität der Messergebnisse über einen breiten Frequenzbereich stark schwanken.

Demzufolge wäre es wünschenswert, ein Verfahren und einen Sensor zum Bestimmen eines die Impedanz einer Suspension anzeigenden Werts bereitzustellen, das/der eine hohe Messgenauigkeit hat und zuverlässige Messungen in einem weiten Frequenzbereich ermöglicht.

Die Erfindung umfasst ein Verfahren zum Bestimmen eines die Impedanz einer Suspension anzeigenden Werts gemäß Anspruch 1 und Anspruch 8 sowie einen Sensor zum Bestimmen eines die Impedanz einer Suspension anzeigenden Werts gemäß Anspruch 12 und Anspruch 16. Weitere Ausführungsformen sind in den abhängigen Ansprüchen enthalten.

Das Verfahren umfasst die Schritte des Bestimmens eines ersten Impedanz-Messwerts auf Basis des Erregerstroms und einer ersten Spannung an einem ersten Paar von Messelektroden und des Bestimmens eines zweiten Impedanz-Messwerts auf Basis der Erregerstroms und einer zweiten Spannung an einem zweiten Paar von Messelektroden. Das erste und das zweite Paar von Messelektroden sind unterschiedliche Paare, d.h. das erste und das zweite Paar von Messelektroden unterscheiden sich zumindest in einer Messelektrode. Das wiederum heißt, dass der verwendete Sensor zumindest drei Messelektroden hat. Die erste Spannung und die zweite Spannung sind somit Messwerte für unterschiedliche Zell-Geometrien, d.h. Messwerte für unterschiedlichen Messzellen in der Suspension. Der Begriff Messzelle bezieht sich dabei auf die Gesamtheit aller Einflüsse der Suspension auf die Anordnung von zwei bestimmten Messelektroden. Die Begriffe erste Spannung und zweite Spannung beziehen sich insbesondere auf eine erste gemessene Spannung und eine zweite gemessene Spannung. Das Bestimmen des ersten Impedanz-Messwerts kann eine erste Spannungsmessung aufweisen, und das Bestimmen des zweiten Impedanz-Messwerts kann eine zweite Spannungsmessung aufweisen.

Der Begriff Suspension beschreibt eine Verteilung von Partikeln in einer Flüssigkeit. Insbesondere bezeichnet der Begriff Suspension eine Suspension mit Partikeln mit einer Impedanz > 0. Bei den Partikeln kann es sich um nicht lebende Teilchen, wie z.B. Kohlepartikel handeln. Es kann sich aber auch um lebende oder zum Teil lebende Teilchen, wie z.B. Zellen handeln. Die Suspension kann eine Zellpopulation sein. Der Begriff Zellpopulation wird hierin für eine Ansammlung von biologischen Zellen in einer Trägerflüssigkeit verwendet. Insbesondere wird der Begriff Zellpopulation für Ansammlungen von biologischen Zellen verwendet, die einen signifikanten Anteil an lebenden Zellen haben. Durch das Bestimmen von einem bzw. mehreren, die Impedanz der Suspension anzeigenden Werten kann auf ein oder mehrere Eigenschaften der Suspension geschlossen werden.

Gemäß einer weiteren Ausführungsform besteht das erste Paar von Messelektroden aus einer ersten Messelektrode und einer zweiten Messelektrode und das zweite Paar von Messelektroden aus der ersten Messelektrode und einer dritten Messelektrode. In anderen Worten, das erste Paar von Messelektroden und das zweite Paar von Messelektroden bestehen aus insgesamt drei Messelektroden. Auf diese Weise können zwei Paare von Messelektroden mit einer minimalen Gesamtanzahl von Messelektroden bereitgestellt werden. Dadurch kann das Verfahren mit einem Sensor mit wenigen Bauteilen durchgeführt werden.

Gemäß einer weiteren Ausführungsform besteht das erste Paar von Messelektroden aus einer ersten Messelektrode und einer zweiten Messelektrode und das zweite Paar von Messelektroden aus einer dritten Messelektrode und einer vierten Messelektrode. Auf diese Weise sind das erste Paar von Messelektroden und das zweite Paar von Messelektroden unabhängig voneinander. Die Messungen an dem ersten Paar von Messelektroden und dem zweiten Paar von Messelektroden können separat voneinander durchgeführt werden. Sie können auch zeitgleich durchgeführt werden, je nach nachgeschalteter Signalverarbeitung. Das Trennen der zwei Paare von Messelektroden kann die nachgeschaltete Signalverarbeitung vereinfachen. Außerdem können die Geometrie der Messzelle des ersten Paars von Messelektroden und die Geometrie der Messzelle des zweiten Paars von Messelektroden einen höheren Grad an Unabhängigkeit aufweisen als in dem vorhin genannten Fall von insgesamt drei Messelektroden. Auf diese Weise kann es möglich sein, Störeinflüsse noch besser aus den Messergebnissen zu entfernen.

Erfindungsgemäß weist das Bestimmen des die Impedanz der Suspension anzeigenden Werts eine Differenzbildung eines ersten angepassten Impedanz-Werts und eines zweiten angepassten Impedanz-Werts auf, wobei der erste angepasste Impedanz-Wert und der zweite angepasste Impedanz-Wert durch Anwenden einer Korrekturfunktion auf den ersten Impedanz-Messwert und den zweiten Impedanz-Messwert erhalten werden. Die Differenzbildung stellt eine wenig komplexe, aber effektive Maßnahme dar, einen signifikanten Anteil der Störeinflüsse auf die Messwerte herauszurechnen. Somit kann mit relativ geringem Rechenaufwand eine starke Verbesserung der Messgenauigkeit des die Impedanz einer Suspension anzeigenden Werts erreicht werden. Die oben genannte Korrekturfunktion kann das Übertragungsverhalten der Messanordnung abbilden. Auf diese Weise kann der Einfluss der Messanordnung auf die gemessenen Signale, wie z.B. durch zusätzliche Signallaufzeiten, Verstärkungen, Verluste, etc., berücksichtigt werden.

Gemäß einer weiteren Ausführungsform weist das Bestimmen des die Impedanz der Suspension anzeigenden Werts eine Differenzbildung eines ersten Geometriefaktors und eines zweiten Geometriefaktors auf, wobei der erste Geometriefaktor die Messgeometrie des ersten Paars von Messelektroden abbildet und wobei der zweite Geometriefaktor die Messgeometrie des zweiten Paars von Messelektroden abbildet. Durch die genannte Differenzbildung können die unterschiedlichen geometrischen Anordnungen der zwei Paare von Messelektroden in einer wenig komplexen, aber effektiven Weise berücksichtigt werden. Der Tatsache, dass durch die Verwendung von unterschiedlichen Paaren von Messelektroden dem ersten Impedanz-Messwert und dem zweiten Impedanz-Messwert unterschiedliche Messzellen zugrunde liegen, kann somit Rechnung getragen werden. Der erste Geometriefaktor und der zweite Geometriefaktor können vor dem Bestimmen des die Impedanz der Suspension anzeigenden Werts berechnet werden. Es ist auch möglich, dass der erste Geometriefaktor und der zweite Geometriefaktor in einer Kalibrierungsphase vor der eigentlichen Durchführung des Verfahrens experimentell bestimmt werden. Der erste und der zweite Geometriefaktor können für das Bestimmen von mehreren, die Impedanz der Suspension anzeigenden Werten im Rahmen einer Impedanzspektroskopie als konstant angenommen werden.

Erfindungsgemäß erfolgt das Bestimmen des die Impedanz der Suspension anzeigenden Werts gemäß folgender Formel:

$$Z = k \frac{1}{\left( \lambda_1 - \lambda_2 \right)} \left( G_{el}^{-1} \left( Z_{sig} \right) \Big|_1 - G_{el}^{-1} \left( Z_{sig} \right) \Big|_2 \right),$$

wobei $Z_{sig}|_1$, den ersten Impedanz-Messwert bezeichnet, $Z_{sig}|_2$ den zweiten Impedanz-Messwert bezeichnet, $G_{el}^{-1}$ eine Korrekturfunktion bezeichnet, die das Übertragungsverhalten der Messanordnung abbildet, $\lambda_1$ einen ersten Geometriefaktor bezeichnet, der die Messgeometrie des ersten Paars von Messelektroden abbildet, $\lambda_2$ einen zweiten Geometriefaktor bezeichnet, der die Messgeometrie des zweiten Paars von Messelektroden abbildet, und k eine Proportionalitätskonstante bezeichnet. Der erste Geometriefaktor $\lambda_1$ und der zweite Geometriefaktor $\lambda_2$ können die Geometrie der oben beschriebenen, unterschiedlichen Messzellen abbilden. Die Korrekturfunktion $G_{el}^{-1}$ ist als inverse Funktion bezeichnet, was durch das hochgestellte -1 angezeigt ist. Diese Schreibweise trägt der Tatsache Rechnung, dass es sich bei $Z_{sig}|_1$ und $Z_{sig}|_2$ um die Impedanz-Messwerte nach Durchlaufen der Messanordnung handelt. Das Übertragungsverhalten der Messanordnung kann durch eine Funktion $G_{el}$ beschrieben werden. Somit erlaubt die Korrekturfunktion $G_{el}^{-1}$ ein Rückrechnen, welche Impedanz-Messwerte an den Messelektroden direkt angelegen haben. Die zu verwendenden Werte für k, $G_{el}^{-1}$, $\lambda_1$ und $\lambda_2$ können berechnet werden oder in einer Kalibrierungsphase bestimmt werden oder teilweise berechnet und teilweise in einer Kalibrierungsphase bestimmt werden.

Gemäß einer weiteren Ausführungsform weist das Verfahren auf: Messen der ersten Spannung an dem ersten Paar von Messelektroden, und Messen der zweiten Spannung an dem zweiten Paar von Messelektroden, wobei das Messen der ersten Spannung und das Messen der zweiten Spannung im Wesentlichen zeitgleich erfolgen. Auf diese Weise ist es möglich, den Einfluss der zeitlichen Variabilität der Störeinflüsse auf die Bestimmung des die Impedanz einer Suspension anzeigenden Werts klein zu halten oder ganz zu eliminieren.

Gemäß einer alternativen Ausführungsform weist das Verfahren auf: Messen der ersten Spannung an dem ersten Paar von Messelektroden, und Messen der zweiten Spannung an dem zweiten Paar von Messelektroden, wobei das Messen der ersten Spannung und das Messen der zweiten Spannung zeitlich versetzt erfolgen. Insbesondere können die an dem ersten Paar von Messelektroden gemessene Spannung und die an dem zweiten Paar von Messelektroden gemessene Spannung nacheinander der nachgelagerten Signalverarbeitung bereitgestellt werden. Dazu können beispielsweise entsprechende Schalter zwischen dem ersten Paar von Messelektroden und dem zweiten Paar von Messelektroden und der nachgelagerten Signalverarbeitung vorgesehen sein. Auf diese Weise kann die nachgelagerte Signalverarbeitung mit vergleichsweise wenigen Bauteilen implementiert werden sowie kompakt und Energie-effizient gestaltet werden.

Unabhängig davon, ob das Messen der ersten Spannung und das Messen der zweiten Spannung im Wesentlichen zeitgleich oder zeitlich versetzt erfolgen, findet das Messen der ersten Spannung sowie das Messen der zweiten Spannung jeweils dann statt, wenn der mit der Erregerfrequenz oszillierende Erregerstrom durch die Suspension erzeugt wird. In anderen Worten, es wird Spannung an den Messelektroden gemessen, während der mit der Erregerfrequenz oszillierende Erregerstrom an der Suspension anliegt. Dementsprechend wird das elektrische Verhalten der Suspension bei Anlegen des mit der Erregerfrequenz oszillierenden Erregerstroms gemessen. Der Erregerstrom kann ebenfalls gemessen werden. Es ist auch möglich, dass der Erregerstrom bekannt ist bzw. als Folge eines bekannten Erzeugungsmechanismus als bekannt angenommen wird.

Gemäß einer weiteren Ausführungsform ist die Erregerfrequenz des Erregerstroms zwischen 50 kHz und 20 MHz. Mit einem Erregerstrom in diesem Frequenzbereich können besonders relevante, die Impedanz einer Zellpopulation anzeigende Werte ermittelt werden, welche insbesondere gut auf die Mengen und/oder die Größe und/oder Homogenität der lebenden Zellen der Zellpopulation schließen lassen. Der genannte Frequenzbereich liegt in dem sogenannten β-Dispersionsgebiet vieler Zellpopulationen, auf das unten nochmals eingegangen wird.

Gemäß einer weiteren Ausführungsform umfasst das Bestimmen des ersten Impedanz-Messwerts und das Bestimmen des zweiten Impedanz-Messwerts: Abtasten des Erregerstroms, Abtasten der ersten Spannung, und Abtasten der zweiten Spannung. Das Abtasten von Erregerstrom, erster Spannung und zweiter Spannung hilft bei dem hochgenauen Bestimmen des die Impedanz einer Suspension anzeigenden Werts über einen weiten Frequenzbereich. Das Abtasten des Erregerstroms, das Abtasten der ersten Spannung und das Abtasten der zweiten Spannung ermöglichen das Erzeugen von Abtastwerten zu genau festgelegten Zeitpunkten. Diese zeitlich diskretisierten Abtastwerte können nach dem Abtasten analysiert und zueinander in Beziehung gesetzt werden, ohne dass die dem Abtasten nachgelagerte Signalverarbeitung echtzeitfähig sein muss. Es kann eine vergleichsweise große, durch das Abtasten in der Zeitdimension klar definierte Datenbasis verwendet werden, um den die Impedanz der Suspension anzeigenden Wert hochgenau zu bestimmen. Gegenüber früheren Ansätzen, die darauf beruhen, charakteristische Eigenschaften einer Suspension durch eine komplizierte analoge Signalverarbeitung zu ermitteln, ermöglicht das Abtasten ein Minimieren der Störeinflüsse nach dem Abtasten, da die Signalverarbeitung der diskretisierten Abtastwerte sehr robust ausgebildet werden kann. Die Störeinflüsse zwischen dem Messen von Erregerstrom, erster Spannung und zweiter Spannung und dem Abtasten von Erregerstrom, erster Spannung und zweiter Spannung können sehr gering gehalten werden. Weiterhin kann das Abtasten des Erregerstroms, der ersten Spannung und der zweiten Spannung auf die Erregerfrequenz abgestimmt sein, wodurch eine hohe Genauigkeit des Abtastens bei den relevanten Frequenzen und eine spektrale Begrenzung der Störeinflüsse ermöglicht werden kann.

Das Abtasten des Erregerstroms, das Abtasten der ersten Spannung und das Abtasten der zweiten Spannung können ein Abtasten von abgeleiteten Werten von Erregerstrom, erster Spannung und zweiter Spannung sein. Beispielsweise kann für den Erregerstrom ein erstes Signal erzeugt werden, welches den Erregerstrom abbildet. Dieses erste Signal kann beispielsweise ein Spannungssignal sein. Das erste Signal kann dann direkt oder nach einer Verstärkung abgetastet werden. Auch eine solche Signalverarbeitung fällt im Sinne des vorliegenden Dokuments unter den Begriff des Abtastens des Erregerstroms. Es ist weiterhin möglich, dass die erste Spannung zwischen dem ersten Paar von Messelektroden in Form eines zweiten Signals abgegriffen wird. Dieses zweite Signal kann auch entweder direkt oder verstärkt abgetastet werden. Wie beim Abtasten des Erregerstroms fällt auch eine solche Vorverarbeitung des zweiten Signals unter den Begriff des Abtastens der ersten Spannung. Es ist weiterhin möglich, dass die zweite Spannung zwischen dem zweiten Paar von Messelektroden in Form eines dritten Signals abgegriffen wird. Dieses dritte Signal kann auch entweder direkt oder verstärkt abgetastet werden. Wie beim Abtasten des Erregerstroms fällt auch eine solche Vorverarbeitung des dritten Signals unter den Begriff des Abtastens der zweiten Spannung.

Gemäß einer weiteren Ausführungsform weist das Verfahren weiterhin die folgenden Schritte auf: Einstellen einer ersten Abtastrate für das Abtasten des Erregerstroms, Einstellen einer zweiten Abtastrate für das Abtasten der ersten Spannung, und Einstellen einer dritten Abtastrate für das Abtasten der zweiten Spannung. Insbesondere kann das Einstellen der ersten Abtastrate und/oder das Einstellen der zweiten Abtastrate und/oder das Einstellen der dritten Abtastrate auf Basis der Erregerfrequenz des Erregerstroms erfolgen. Dabei können die erste Abtastrate, die zweite Abtastrate und die dritte Abtastrate gleich sein oder verschieden sein. Das Einstellen der ersten Abtastrate, das Einstellen der zweiten Abtastrate und das Einstellen der dritten Abtastrate erlauben ein Anpassen des Bestimmens des die Impedanz der Suspension anzeigenden Werts an die Rahmenbedingungen eines vorliegenden Messvorgangs, insbesondere an die Erregerfrequenz des Erregerstroms für den vorliegenden Messvorgang. Auf diese Weise ist es möglich, für jeden Messvorgang optimierte Abtastraten einzusetzen, insbesondere eine bezüglich Genauigkeit und/oder Signalverarbeitungskomplexität optimierte Abtastrate einzustellen. Die erste Abtastrate, die zweite Abtastrate und die dritte Abtastrate werden für das Abtasten des Erregerstroms, für das Abtasten der ersten Spannung und für das Abtasten der zweiten Spannung verwendet. Demzufolge werden die erste Abtastrate, die zweite Abtastrate und die dritte Abtastrate vor dem Abtasten des Erregerstroms, der ersten Spannung und der zweiten Spannung eingestellt. Die Formulierung des Einstellens der ersten Abtastrate und des Einstellens der zweiten Abtastrate und des Einstellens der dritten Abtastrate umfasst auch das Einstellen einer Abtastrate und das Verwenden dieser einen Abtastrate als erste Abtastrate, als zweite Abtastrate und als dritte Abtastrate.

Gemäß einer weiteren Ausführungsform werden die erste Abtastrate, die zweite Abtastrate und die dritte Abtastrate mindestens auf das 4-fache der Erregerfrequenz des Erregerstroms, insbesondere im Wesentlichen auf das 4-fache der Erregerfrequenz des Erregerstroms, eingestellt. Durch das Verwenden von dem mindestens 4-fachen der Erregerfrequenz des Erregerstroms für das Abtasten von Erregerstrom, erster Spannung und zweiter Spannung wird sichergestellt, dass Erregerstrom, erste Spannung und zweite Spannung sehr genau abgetastet werden und dass keine Signalinformation um die Erregerfrequenz herum verloren geht. Das Abtasttheorem ist mit beruhigendem Abstand übererfüllt. Insbesondere können die erste Abtastrate, die zweite Abtastrate und die dritte Abtastrate im Wesentlichen auf das 4-fache der Erregerfrequenz des Erregerstroms oder sogar genau auf das 4-fache der Erregerfrequenz des Erregerstroms eingestellt werden.

Gemäß einer weiteren Ausführungsform weist der Schritt des Bestimmens des ersten Impedanz-Messwerts ein Durchführen einer ersten komplexen Fourier-Transformation auf Basis der Abtastwerte des Erregerstroms und der Abtastwerte der ersten Spannung auf und der Schritt des Bestimmens des zweiten Impedanz-Messwerts ein Durchführen einer zweiten komplexen Fourier-Transformation auf Basis der Abtastwerte des Erregerstroms und der Abtastwerte der zweiten Spannung auf. Dabei kann insbesondere eine komplexe diskrete Fourier-Transformation zum Einsatz kommen. Die Abtastwerte des Erregerstroms, die Abtastwerte der ersten Spannung und die Abtastwerte der zweiten Spannung können als jeweilige Realteile eines komplexen Strom- bzw. Spannungssignals angesehen werden. Mittels komplexer Fourier-Transformation, welche abgetastete Strom- und Spannungswerte gemeinsam berücksichtigt, kann die komplexe Impedanz zwischen Erregerstrom und erster Spannung bzw. zwischen Erregerstrom und zweiter Spannung bestimmt werden. Insbesondere können dabei komplexe Impedanzen bei der Erregerfrequenz bestimmt werden, welche die Grundlage für den ersten Impedanz-Messwert bzw. den zweiten Impedanz-Messwert darstellen können.

Gemäß einer weiteren Ausführungsform weist das Verfahren weiterhin auf: Bestimmen eines dritten Impedanz-Messwerts auf Basis des Erregerstroms und einer dritten Spannung an einem dritten Paar von Messelektroden, Bestimmen des die Impedanz der Suspension anzeigenden Werts durch In-Beziehung-Setzen des ersten Impedanz-Messwerts, des zweiten Impedanz-Messwerts und des dritten Impedanz-Messwerts. Durch das Bestimmen eines dritten Impedanz-Messwerts und das In-Beziehung-Setzen des ersten, zweiten und dritten Impedanz-Messwerts können Störeinflüsse in einem noch höheren Maß aus den Messergebnissen entfernt werden. Insbesondere können durch die Verwendung von drei Impedanz-Messwerten von drei unterschiedlichen Paaren von Messelektroden die Störeinflüsse einer Ordnung höher reduziert oder sogar eliminiert werden, als dies bei der Verwendung von zwei unterschiedlichen Paaren von Messelektroden gelingt. Die drei Paare von Messelektroden bilden drei unterschiedliche geometrische Anordnungen in der Suspension. Somit stehen Messwerte für drei Messzellen zur Verfügung. Die drei Paare von Messelektroden können von insgesamt vier oder mehr Messelektroden gebildet werden. Insbesondere ist es möglich, dass insgesamt vier, fünf oder sechs Messelektroden verwendet werden, aus denen drei unterschiedliche Paare von Messelektroden zur jeweiligen Bestimmung eines Impedanz-Messwerts herangezogen werden.

Gemäß einer weiteren Ausführungsform weist das Bestimmen des die Impedanz der Suspension anzeigenden Werts eine erste Differenzbildung des ersten Impedanz-Messwerts und des zweiten Impedanz-Messwerts und eine zweite Differenzbildung des ersten Impedanz-Messwerts und des dritten Impedanz-Messwerts und eine dritte Differenzbildung des zweiten Impedanz-Messwerts und des dritten Impedanz-Messwerts auf. Analog zu dem oben beschriebenen Fall von zwei Paaren von Messelektroden stellen die erste, zweite und dritte Differenzbildung wenig komplexe, aber effektive Maßnahmen dar, einen signifikanten Anteil der Störeinflüsse auf die Messwerte herauszurechnen.

Gemäß einer alternativen Ausführungsform weist das Bestimmen des die Impedanz der Suspension anzeigenden Werts eine erste Differenzbildung eines ersten angepassten Impedanz-Werts und eines zweiten angepassten Impedanz-Werts und eine zweite Differenzbildung des ersten angepassten Impedanz-Werts und eines dritten angepassten Impedanz-Werts und eine dritte Differenzbildung des zweiten angepassten Impedanz-Werts und des dritten angepassten Impedanz-Werts auf, wobei der erste angepasste Impedanz-Wert, der zweite angepasste Impedanz-Wert und der dritte angepasste Impedanz-Wert durch Anwenden einer Korrekturfunktion auf den ersten Impedanz-Messwert, den zweiten Impedanz-Messwert und den dritten Impedanz-Messwert erhalten werden. Gemäß einer weiteren Ausführungsform kann die Korrekturfunktion das Übertragungsverhalten der Messanordnung abbilden.

Gemäß einer weiteren Ausführungsform weist das Bestimmen des die Impedanz der Suspension anzeigenden Werts eine erste Differenzbildung eines ersten Geometriefaktors und eines zweiten Geometriefaktors und eine zweite Differenzbildung des ersten Geometriefaktors und eines dritten Geometriefaktors und eine dritte Differenzbildung des zweiten Geometriefaktors und des dritten Geometriefaktors auf, wobei der erste Geometriefaktor die Messgeometrie des ersten Paars von Messelektroden abbildet, der zweite Geometriefaktor die Messgeometrie des zweiten Paars von Messelektroden abbildet und der dritte Geometriefaktor die Messgeometrie des dritten Paars von Messelektroden abbildet. Durch die genannten Differenzbildungen können die unterschiedlichen geometrischen Anordnungen der drei Paare von Messelektroden in einer wenig komplexen, aber effektiven Weise berücksichtigt werden.

Gemäß einer weiteren Ausführungsform erfolgt das Bestimmen des die Impedanz der Suspension anzeigenden Werts gemäß folgender Formel:

$$Z^2 = k_2 \frac{\lambda_3\left(G_{el}^{-1}\left(Z_{sig}\right)\big|_2 - G_{el}^{-1}\left(Z_{sig}\right)\big|_1\right)}{(\lambda_1-\lambda_2)(\lambda_1-\lambda_3)(\lambda_2-\lambda_3)} +$$

$$+ k_2 \frac{\lambda_2\left(G_{el}^{-1}\left(Z_{sig}\right)\big|_1 - G_{el}^{-1}\left(Z_{sig}\right)\big|_3\right)}{(\lambda_1-\lambda_2)(\lambda_1-\lambda_3)(\lambda_2-\lambda_3)} +$$

$$+ k_2 \frac{\lambda_1\left(G_{el}^{-1}\left(Z_{sig}\right)\big|_3 - G_{el}^{-1}\left(Z_{sig}\right)\big|_2\right)}{(\lambda_1-\lambda_2)(\lambda_1-\lambda_3)(\lambda_2-\lambda_3)} ,$$

wobei $Z_{sig}|_1$ den ersten Impedanz-Messwert bezeichnet, $Z_{sig}|_2$ den zweiten Impedanz-Messwert bezeichnet, $Z_{sig}|_3$ den dritten Impedanz-Messwert bezeichnet, $G_{el}^{-1}$ eine Korrekturfunktion bezeichnet, die das Übertragungsverhalten der Messanordnung abbildet, $\lambda_1$ einen ersten Geometriefaktor bezeichnet, der die Messgeometrie des ersten Paars von Messelektroden abbildet, $\lambda_2$ einen zweiten Geometriefaktor bezeichnet, der die Messgeometrie des zweiten Paars von Messelektroden abbildet, $\lambda_3$ einen dritten Geometriefaktor bezeichnet, der die Messgeometrie des dritten Paars von Messelektroden abbildet, und $k_2$ eine Proportionalitätskonstante bezeichnet. Die zu verwendenden Werte für $k_2$, $G_{el}^{-1}$, $\lambda_1$, $\lambda_2$ und $\lambda_3$ können berechnet werden oder in einer Kalibrierungsphase bestimmt werden oder teilweise berechnet und teilweise in einer Kalibrierungsphase bestimmt werden.

Gemäß einer weiteren Ausführungsform erfolgt das Bestimmen des die Impedanz der Suspension anzeigenden Werts gemäß folgender Formel:

$$Z = k_2 \frac{\lambda_3\left(G_{el}^{-1}\left(Z_{sig}\right)\big|_2 - G_{el}^{-1}\left(Z_{sig}\right)\big|_1\right)}{(\lambda_1-\lambda_2)(\lambda_1-\lambda_3)(\lambda_2-\lambda_3)} +$$

$$+ k_2 \frac{\lambda_2\left(G_{el}^{-1}\left(Z_{sig}\right)\big|_1 - G_{el}^{-1}\left(Z_{sig}\right)\big|_3\right)}{(\lambda_1-\lambda_2)(\lambda_1-\lambda_3)(\lambda_2-\lambda_3)} +$$

$$+ k_2 \frac{\lambda_1\left(G_{el}^{-1}\left(Z_{sig}\right)\big|_3 - G_{el}^{-1}\left(Z_{sig}\right)\big|_2\right)}{(\lambda_1-\lambda_2)(\lambda_1-\lambda_3)(\lambda_2-\lambda_3)} ,$$

wobei $Z_{sig}|_1$ den ersten Impedanz-Messwert bezeichnet, $Z_{sig}|_2$ den zweiten Impedanz-Messwert bezeichnet, $Z_{sig}|_3$ den dritten Impedanz-Messwert bezeichnet, $G_{el}^{-1}$ eine Korrekturfunktion bezeichnet, die das Übertragungsverhalten der Messanordnung abbildet, $\lambda_2$ einen ersten Geometriefaktor bezeichnet, der die Messgeometrie des ersten Paars von Messelektroden abbildet, $\lambda_2$ einen zweiten Geometriefaktor bezeichnet, der die Messgeometrie des zweiten Paars von Messelektroden abbildet, $\lambda_3$ einen dritten Geometriefaktor bezeichnet, der die Messgeometrie des dritten Paars von Messelektroden abbildet, und $k_2$ eine Proportionalitätskonstante bezeichnet. Die zu verwendenden Werte für $k_2$, $G_{el}^{-1}$, $\lambda_1$, $\lambda_2$ und $\lambda_3$ können berechnet werden oder in einer Kalibrierungsphase bestimmt werden oder teilweise berechnet und teilweise in einer Kalibrierungsphase bestimmt werden.

Alternativ umfasst die Erfindung ein Verfahren zum Bestimmen eines die Impedanz einer Suspension anzeigenden Werts im Rahmen einer Impedanzspektroskopie, aufweisend die folgenden Schritte: Erzeugen einer mit einer Erregerfrequenz oszillierenden, an der Suspension anliegenden Erregerspannung; Bestimmen eines ersten Impedanz-Messwerts auf Basis der Erregerspannung und eines ersten Stroms durch ein erstes Paar von Messelektroden;

Bestimmen eines zweiten Impedanz-Messwerts auf Basis der Erregerspannung und eines zweiten Stroms durch ein zweites Paar von Messelektroden; Bestimmen des die Impedanz der Suspension anzeigenden Werts durch In-Beziehung-Setzen des ersten Impedanz-Messwerts und des zweiten Impedanz-Messwerts. Das Erzeugen einer Erregerspannung und das Bestimmen eines ersten und zweiten Impedanz-Messwerts auf Basis der Erregerspannung und des ersten und zweiten Stroms stellt eine alternative Ausführungsform zu dem oben beschriebenen Erzeugen eines Erregerstroms und dem Bestimmen eines ersten und zweiten Impedanz-Messwerts auf Basis des Erregerstroms und der ersten und zweiten Spannung dar. In anderen Worten, alternative Ausführungsformen der Erfindung bestehen darin, eine Erregerspannung an die Suspension anzulegen und Stromflüsse durch Messelektroden für die Bestimmung der Impedanz-Messwerte heranzuziehen. Beispielsweise ist es möglich, die Erregerspannung so anzulegen, dass kein Stromfluss durch ein Paar von Erregerelektroden fließt. In anderen Worten, das Paar von Erregerelektroden kann lediglich dazu verwendet werden, für eine zeitlich veränderliches Potentialdifferenz in der Suspension zu sorgen. Weiterhin können das erste Paar von Messelektroden und das zweite Paar von Messelektroden beispielsweise jeweils über einen Messwiderstand oder über einen Messkondensator miteinander verbunden sein. Somit ist jeweils durch den Messwiderstand / Messkondensator und die Suspension ein geschlossener Wechselstromkreis vorhanden, und die Spannung an dem Messwiderstand / Messkondensator kann als Maß für den Strom durch das jeweilige Paar von Messelektroden abgegriffen werden. Die zusätzlichen Merkmale, Modifikationen und technischen Effekte, die oben mit Bezug auf das Verfahren unter Verwendung eines Erregerstroms beschrieben worden sind, gelten für das Verfahren unter Verwendung einer Erregerspannung analog und sind hiermit explizit für diese alternative Lösung offenbart.

Beispielhafte Ausführungsformen der Erfindung umfassen weiterhin ein Verfahren zum Herleiten von mindestens einer charakteristischen Eigenschaft einer Suspension, aufweisend die folgenden Schritte: mehrmaliges Durchführen des Verfahrens zum Bestimmen eines die Impedanz einer Suspension anzeigenden Werts gemäß einer der oben beschriebenen Ausführungsformen, wobei bei dem mehrmaligen Durchführen des Verfahrens eine Mehrzahl von unterschiedlichen Erregerfrequenzen verwendet wird und eine Mehrzahl von die Impedanz der Suspension anzeigenden Werten für die Mehrzahl von unterschiedlichen Erregerfrequenzen bestimmt wird; Herleiten einer Mehrzahl von die Permittivität der Suspension anzeigenden Werten auf Basis der Mehrzahl von die Impedanz der Suspension anzeigenden Werten; und Herleiten der mindestens einen charakteristischen Eigenschaft der Suspension durch In-Beziehung-Setzen der Mehrzahl von die Permittivität der Suspension anzeigenden Werten. Dabei kann das In-Beziehung-Setzen das Bilden einer Differenz zwischen zwei die Permittivität der Suspension anzeigenden Werten beinhalten und/oder das Bestimmen der Steigung einer Kurve beinhalten, die durch die Mehrzahl von die Permittivität der Suspension anzeigenden Werten gelegt ist, und/oder das Bestimmen eines Wendepunkts einer Kurve beinhalten, die durch die Mehrzahl von die Permittivität der Suspension anzeigenden Werten gelegt ist, und/oder das Bestimmen von weiteren charakteristischen Eigenschaften der Mehrzahl von die Permittivität der Suspension anzeigenden Werten beinhalten. Über diese charakteristischen Eigenschaften der Menge an gewonnen Werten kann auf charakteristische Eigenschaften der Suspension, insbesondere auf charakteristische Eigenschaften einer Zellpopulation, rückgeschlossen werden. Durch das hochgenaue Bestimmen der Mehrzahl von die Permittivität der Suspension anzeigenden Werten über einen vergleichsweise großen Frequenzbereich können auch die charakteristischen Eigenschaften der Suspension mit hoher Genauigkeit bestimmt werden. Die Mehrzahl der die Permittivität der Suspension anzeigenden Werte können Kapazitätswerte oder Permittivitätswerte sein.

Gemäß einer weiteren Ausführungsform wird das genannte Verfahren zum Bestimmen eines die Impedanz einer Suspension anzeigenden Werts für zwischen 2 und 50 unterschiedliche Erregerfrequenzen durchgeführt. Insbesondere kann das Verfahren zum Bestimmen eines die Impedanz einer Suspension anzeigenden Werts für zwischen 10 und 40 unterschiedliche Erregerfrequenzen, weiter insbesondere für zwischen 20 und 30 unterschiedliche Erregerfrequenzen durchgeführt werden. Es hat sich gezeigt, dass für die genannte Anzahl von Durchläufen des Verfahrens und die entsprechende Anzahl von die Impedanz der Suspension anzeigenden Werten, insbesondere bei zwischen 10 und 40 unterschiedlichen Erregerfrequenzen bzw. weiter insbesondere bei zwischen 20 und 30 unterschiedlichen Erregerfrequenzen, ein guter Kompromiss zwischen Aufwendigkeit des Verfahrens zum Herleiten von mindestens einer charakteristischen Eigenschaft der Suspension und Genauigkeit der Ergebnisse bezüglich der mindestens einen charakteristischen Eigenschaft der Suspension erreicht werden kann. Insbesondere können für zwischen 10 und 40 unterschiedliche Erregerfrequenzen bzw. weiter insbesondere für zwischen 20 und 30 unterschiedliche Erregerfrequenzen besonders aussagekräftige Kurven der die Permittivität der Suspension anzeigenden Werte, insbesondere der Permittivität selbst, erstellt werden. Die genannte Anzahl an Werten erlaubt das Erstellen von Verläufen gegenüber der Erregerfrequenz mit ausreichender Genauigkeit, um charakteristische Eigenschaften wie Steigung und Wendepunkt mit hoher Genauigkeit zu bestimmen. Diese Ausführungen gelten insbesondere für das Herleiten von mindestens einer charakteristischen Eigenschaft einer Zellpopulation.

Gemäß einer weiteren Ausführungsform stammen die unterschiedlichen Erregerfrequenzen aus einem Frequenzbereich von 100 kHz bis 10 MHz. Die Ausdrucksweise, dass die unterschiedlichen Erregerfrequenzen aus dem genannten Frequenzbereich stammen, bedeutet, dass mindestens der genannte Frequenzbereich von den unterschiedlichen Erregerfrequenzen abgedeckt ist. Dies wiederum bedeutet, dass die niedrigste Erregerfrequenz 100 kHz oder weniger beträgt und dass die höchste Erregerfrequenz 10 MHz oder mehr beträgt. In anderen Worten, die niedrigste Erregerfrequenz und die höchste Erregerfrequenz bilden eine dazwischen liegende Erregerfrequenz-Spanne, die mindestens den Bereich zwischen 100 kHz bis 10 MHz beinhaltet. Mit den die Impedanz der Suspension anzeigenden Werten für die Erregerfrequenzen von 100 kHz bis 10 MHz können ein oder mehrere charakteristische Eigenschaften von einer Vielzahl von Suspensionen, insbesondere von einer Vielzahl von Zellpopulationen, mit hoher Genauigkeit bestimmt werden.

In einer weiteren Ausführungsform stammen die unterschiedlichen Erregerfrequenzen aus einem Frequenzbereich von 50 kHz bis 20 MHz. Dadurch kann das Herleiten der mindestens einen charakteristischen Eigenschaft der Suspension nochmals verfeinert werden. Dies gilt insbesondere für das Herleiten der mindestens einen charakteristischen Eigenschaft einer Zellpopulation. Beispielhafte Ausführungsformen des oben beschriebenen Verfahrens zum Bestimmen eines die Impedanz einer Suspension anzeigenden Werts erlauben auf Grund der erhöhten Messgenauigkeit und/oder der erhöhten Zuverlässigkeit der Messergebnisse eine Erweiterung des Frequenzbereichs der Impedanzspektroskopie und somit eine umfassendere Bestimmung von einer oder mehreren charakteristischen Eigenschaften der Suspension, ohne auf zusätzliche und aufwendigere Verfahren zurückgreifen zu müssen. Es ist auch möglich, dass das Verfahren auf einen erweiterten Bereich von Suspensionen, insbesondere auf einen erweiterten Bereich von Zellpopulationen, angewendet werden kann.

Gemäß einer weiteren Ausführungsform beinhaltet das Herleiten der mindestens einen charakteristischen Eigenschaft der Suspension ein Erstellen eines Verlaufs der die Permittivität der Suspension anzeigenden Werte über die unterschiedlichen Erregerfrequenzen. In anderen Worten, es kann eine Kurve durch die die Permittivität der Suspension anzeigenden Werte gegenüber der Erregerfrequenz gelegt werden. Dabei kann ein sogenanntes Cole-Cole-Fitting angewendet werden. Aus der resultierenden Kurve bzw. aus dem resultierenden Verlauf können dann Charakteristika wie z.B. Differenzen zwischen Endwerten, Steigungen und Wendepunkte ermittelt werden. Die die Permittivität der Suspension anzeigenden Werte können direkt die bestimmten Werte oder kalibrierte Versionen der bestimmten Werte sein.

Gemäß einer weiteren Ausführungsform ist die Suspension eine Zellpopulation. In einer weiteren Ausführungsform umfasst die mindestens eine charakteristische Eigenschaft der Zellpopulation mindestens eine Eigenschaft von Anzahl der lebenden Zellen, Größe der Zellen und Homogenität der Zellen.

Die gegenwärtige Erfindung umfasst weiterhin einen Sensor zum Bestimmen eines die Impedanz einer Suspension anzeigenden Werts, aufweisend: eine Oszillatorschaltung; ein Paar von Erregerelektroden, die mit der Oszillatorschaltung gekoppelt sind, wobei mittels der Oszillatorschaltung über das Paar von Erregerelektroden ein mit einer Erregerfrequenz oszillierender Erregerstrom durch die Suspension erzeugbar ist; mindestens drei Messelektroden zum Messen einer ersten Spannung in der Suspension zwischen einem ersten Paar der mindestens drei Messelektroden und einer zweiten Spannung in der Suspension zwischen einem zweiten Paar der mindestens drei Messelektroden; und eine Datenverarbeitungseinrichtung, die konfiguriert ist, einen ersten Impedanz-Messwert auf Basis des Erregerstroms und der ersten Spannung zu bestimmen, einen zweiten Impedanz-Messwert auf Basis des Erregerstroms und der zweiten Spannung zu bestimmen, und den die Impedanz der Suspension anzeigenden Wert durch In-Beziehung-Setzen des ersten Impedanz-Messwerts und des zweiten Impedanz-Messwerts zu bestimmen. Die zusätzlichen Merkmale, Modifikationen und technischen Effekte, die oben mit Bezug auf das Verfahren zum Bestimmen eines die Impedanz einer Suspension anzeigenden Werts beschrieben worden sind, gelten für den Sensor zum Bestimmen eines die Impedanz einer Suspension anzeigenden Werts analog.

Gemäß einer weiteren Ausführungsform sind die mindestens drei Messelektroden zwischen dem Paar von Erregerelektroden angeordnet. Auf diese Weise liegt der Erregerstrom in hoher Stärke entlang den Messelektroden an, und das Verhältnis von Nutzsignal zu Störeinflüssen ist hoch im Vergleich zu anderen geometrischen Anordnungen. Außerdem ermöglicht eine solche Anordnung eine kompakte Bauart des Sensors.

Gemäß einer weiteren Ausführungsform besteht das erste Paar der mindestens drei Messelektroden aus einer ersten Messelektrode und einer zweiten Messelektrode und das zweite Paar der mindestens drei Messelektroden aus der ersten Messelektrode und einer dritten Messelektrode.

Gemäß einer alternativen Ausführungsform sind die mindestens drei Messelektroden mindestens vier Messelektroden, wobei das erste Paar der mindestens vier Messelektroden aus einer ersten Messelektrode und einer zweiten Messelektrode besteht und wobei das zweite Paar der mindestens vier Messelektroden aus einer dritten Messelektrode und einer vierten Messelektrode besteht.

Die oben mit Bezug auf das Verfahren zum Bestimmen eines die Impedanz einer Suspension anzeigenden Werts gemachten Ausführungen zu den verschiedenen Anzahlen von Messelektroden gelten für den Sensor zum Bestimmen eines die Impedanz einer Suspension anzeigenden Werts analog.

Gemäß einer weiteren Ausführungsform sind die dritte und vierte Messelektrode zwischen der ersten und zweiten Messelektrode angeordnet. Eine solche Anordnung ermöglicht eine starke Überlappung der Messzellen. Dadurch besteht eine hohe Wahrscheinlichkeit, dass sich Störeinflüsse in sehr ähnlicher oder gleicher Weise auf die beiden Messzellen auswirken. Dies wiederum erlaubt eine möglichst gute Entfernung der Störeinflüsse aus den Messwerten durch das oben im Detail diskutierte In-Beziehung-Setzen des ersten Impedanz-Messwerts und des zweiten Impedanz-Messwerts. Außerdem ermöglicht eine solche Anordnung eine kompakte Bauart des Sensors.

Gemäß einer weiteren Ausführungsform sind die dritte und vierte Messelektrode an einer anderen Seite des Sensors als die erste und zweite Messelektrode angeordnet. Auf diese Weise kann der gegenseitige Einfluss der Messelektroden klein gehalten werden. Eine weitgehend unabhängige Bestimmung des ersten Impedanz-Messwerts und des zweiten Impedanz-Messwerts wird somit ermöglicht. Abhängig von den Rahmenbedingungen, d.h. abhängig von der zu untersuchenden Suspension und den vorliegenden Störeinflüssen, können auf diese Weise im Einzelfall besonders gute Messergebnisse erzielt werden.

Gemäß einer weiteren Ausführungsform ist die Datenverarbeitungseinrichtung konfiguriert, den die Impedanz der Suspension anzeigenden Wert mittels einer Differenzbildung des ersten Impedanz-Messwerts und des zweiten Impedanz-Messwerts zu bestimmen.

Gemäß einer alternativen Ausführungsform ist die Datenverarbeitungseinrichtung konfiguriert, den die Impedanz der Suspension anzeigenden Wert mittels einer Differenzbildung eines ersten angepassten Impedanz-Werts und eines zweiten angepassten Impedanz-Werts zu bestimmen, wobei die Datenverarbeitungseinrichtung konfiguriert ist, den ersten angepassten Impedanz-Wert und den zweiten angepassten Impedanz-Wert durch Anwenden einer Korrekturfunktion auf den ersten Impedanz-Messwert und den zweiten Impedanz-Messwert zu erhalten. In einer weiteren Ausführungsform bildet die Korrekturfunktion das Übertragungsverhalten der Messanordnung ab.

Gemäß einer weiteren Ausführungsform ist die Datenverarbeitungseinrichtung konfiguriert, den die Impedanz der Suspension anzeigenden Werts mittels einer Differenzbildung eines ersten Geometriefaktors und eines zweiten Geometriefaktors zu bestimmen, wobei der erste Geometriefaktor die Messgeometrie des ersten Paars der mindestens drei Messelektroden abbildet und wobei der zweite Geometriefaktor die Messgeometrie des zweiten Paars der mindestens drei Messelektroden abbildet.

Gemäß einer weiteren Ausführungsform ist die Datenverarbeitungseinrichtung konfiguriert, den die Impedanz der Suspension anzeigenden Wert gemäß folgender Formel zu bestimmen:

$$Z = k \frac{1}{(\lambda_1 - \lambda_2)} \left( G_{el}^{-1}(Z_{sig})\big|_1 - G_{el}^{-1}(Z_{sig})\big|_2 \right),$$

wobei $Z_{sig}|_1$ den ersten Impedanz-Messwert bezeichnet, $Z_{sig}|_2$ den zweiten Impedanz-Messwert bezeichnet, $G_{el}^{-1}$ eine Korrekturfunktion bezeichnet, die das Übertragungsverhalten der Messanordnung abbildet, $\lambda_1$ einen ersten Geometriefaktor bezeichnet, der die Messgeometrie des ersten Paars der mindestens drei Messelektroden abbildet, $\lambda_2$ einen zweiten Geometriefaktor bezeichnet, der die Messgeometrie des zweiten Paars der mindestens drei Messelektroden abbildet, und k eine Proportionalitätskonstante bezeichnet.

Gemäß einer weiteren Ausführungsform ist die Oszillatorschaltung eingerichtet, die Erregerfrequenz in einem Frequenzbereich von 100 kHz bis 10 MHz einzustellen. Das heißt, dass die Oszillatorschaltung in der Lage ist, die Erregerfrequenz in einem Frequenzbereich von 100 kHz bis 10 MHz einzustellen. Durch diese Formulierung ist nicht ausgeschlossen, dass die Oszillatorschaltung die Erregerfrequenz über den angegebenen Frequenzbereich hinaus einstellen kann. Vielmehr bedeutet die Formulierung, dass die Oszillatorschaltung imstande ist, die Erregerfrequenz mindestens in dem Frequenzbereich von 100 kHz bis 10 MHz einzustellen. Insbesondere kann die Oszillatorschaltung eingerichtet sein, die Erregerfrequenz mindestens in einem Frequenzbereich von 50 kHz bis 20 MHz einzustellen.

Gemäß einer weiteren Ausführungsform weist der Sensor weiterhin auf: eine erste Abtastschaltung, die mit mindestens einer von dem Paar von Erregerelektroden gekoppelt ist und im Betrieb Abtastwerte für den Erregerstrom bereitstellt; und mindestens eine weitere Abtastschaltung, die mit den mindestens drei Messelektroden gekoppelt ist und im Betrieb Abtastwerte für die erste Spannung und die zweite Spannung bereitstellt; wobei die Datenverarbeitungseinrichtung mit der ersten Abtastschaltung und der mindestens einen weiteren Abtastschaltung gekoppelt ist. Für die Datengewinnung für den ersten Impedanz-Messwert bzw. für den zweiten Impedanz-Messwert arbeiten die erste Abtastschaltung und die benötigte weitere Abtastschaltung gleichzeitig, d.h. sie erzeugen die Abtastwerte für den Erregerstrom und die Abtastwerte für die erste Spannung bzw. für die zweite Spannung im gleichen Zeitraum. Weiterhin arbeiten die erste Abtastschaltung und die benötigte weitere Abtastschaltung im Betrieb dann, wenn der Erregerstrom anliegt, d.h. die erste Abtastschaltung und die benötigte weitere Abtastschaltung arbeiten im gleichen Zeitraum wie die Oszillatorschaltung.

Der Begriff gekoppelt wird hierin verwendet, um anzuzeigen, dass ein Signal oder eine elektrische Größe von einer Entität zur anderen übertragen werden kann, d.h. dass irgendeine Art von Verbindung zwischen den Entitäten besteht. Es können allerdings andere Komponenten, wie z.B. Verstärker, Transformatoren oder andere elektrische Bauelemente, zwischengeschaltet sein.

Die erste Abtastschaltung kann entweder mit der ersten Erregerelektrode oder mit der zweiten Erregerelektrode gekoppelt sein. Sie kann auch mit beiden Erregerelektroden gekoppelt sein. Generell kann die erste Abtastschaltung in jeder geeigneten Art und Weise derart mit einer oder beiden Erregerelektroden gekoppelt sein, dass ein Messen des Erregerstroms möglich ist.

Gemäß einer weiteren Ausführungsform sind die erste Abtastschaltung und die mindestens eine weitere Abtastschaltung bezüglich ihrer Abtastzeitpunkte synchronisiert.

Gemäß einer weiteren Ausführungsform sind die erste Abtastschaltung und die mindestens eine weitere Abtastschaltung mit der Datenverarbeitungseinrichtung über einen Datenspeicher gekoppelt, wobei der Datenspeicher insbesondere eine Datenaufnahmerate von mindestens 1 Gbit/s hat. Die Datenaufnahmerate von mindestens 1 Gbit/s beschreibt eine mögliche Datenaufnahmerate von mindestens 1 Gbit/s. Die Daten müssen von dem Datenspeicher nicht in dieser Geschwindigkeit aufgenommen werden. Da die Abtastfrequenz von der Erregerfrequenz abhängen kann, können sich im Betrieb für verschiedene Erregerfrequenzen verschiedene Datenaufnahmeraten an dem Datenspeicher ergeben.

Gemäß einer weiteren Ausführungsform ist die Datenverarbeitungseinrichtung eingerichtet, den ersten Impedanz-Messwert mittels einer ersten komplexen Fourier-Transformation auf Basis der Abtastwerte des Erregerstroms und der Abtastwerte der ersten Spannung zu bestimmen und den zweiten Impedanz-Messwert mittels einer zweiten komplexen Fourier-Transformation auf Basis der Abtastwerte des Erregerstroms und der Abtastwerte der zweiten Spannung zu bestimmen.

Gemäß einer weiteren Ausführungsform ist die erste Abtastschaltung über eine erste Verstärkerschaltung mit der mindestens einen von dem Paar von Erregerelektroden gekoppelt und die mindestens eine weitere Abtastschaltung über mindestens eine weitere Verstärkerschaltung mit den mindestens drei Messelektroden gekoppelt.

Gemäß einer weiteren Ausführungsform ist ein Messelement, insbesondere ein Messwiderstand, mit der mindestens einen von dem Paar von Erregerelektroden gekoppelt, und die erste Abtastschaltung ist eingerichtet, die Abtastwerte für den Erregerstrom auf Basis des Spannungsabfalls an dem Messelement bereitzustellen. Aus Symmetriegründen können beide Erregerelektroden jeweils mit einem Messelement, insbesondere mit einem Messwiderstand, gekoppelt sein, auch wenn die erste Abtastschaltung nur mit einem der Messelemente gekoppelt ist.

Gemäß einer weiteren Ausführungsform ist die Oszillatorschaltung über einen Transformator mit dem Paar von Erregerelektroden gekoppelt. So kann eine galvanische Entkopplung zwischen der Oszillatorschaltung und den Erregerelektroden erreicht werden.

Gemäß einer weiteren Ausführungsform hat der Transformator eine parallele Kapazität von 0,5 pF bis 10 pF, insbesondere eine parallele Kapazität von 1 pF bis 5 pF. Die parallele Kapazität kann ein diskretes Bauteil, wie beispielsweise ein parallel zu dem Transformator angeordneter Kondensator, sein. Es ist aber auch möglich, dass die parallele Kapazität eine parasitäre Kapazität des Transformators ist, wobei der Transformator so ausgeführt ist, dass die parallele Kapazität in dem angegebenen Wertebereich liegt. Der Begriff parallele Kapazität bezeichnet eine Kopplungskapazität zwischen der Primärseite und der Sekundärseite des Transformators. Mit einer parallelen Kapazität kann der Einfluss von störenden Kopplungskapazitäten, wie sie zum Beispiel zwischen den Elektroden und der Wand eines Behälters für die Suspension oder zwischen den Elektroden und anderen vorhandenen Sensoren vorliegen können, gering gehalten werden. Da bei Vorliegen der zu dem Transformator parallelen Kapazität und anderen Kopplungskapazitäten oft die kleinere Kapazität bestimmend ist, kann der Einfluss einer unerwünschten störenden Kopplungskapazität auf die Größe der parallelen Kapazität reduziert werden.

Gemäß einer weiteren Ausführungsform weist der Sensor weiterhin eine Steuereinheit auf, die mit der Oszillatorschaltung gekoppelt ist und die Oszillatorschaltung im Betrieb veranlasst, nacheinander mit unterschiedlichen Erregerfrequenzen oszillierende Erregerströme durch die Suspension zu erzeugen. So kann die Steuereinheit verschiedene Messdurchgänge anstoßen, mittels derer eine Impedanzspektroskopie für die Suspension durchgeführt werden kann. Weiterhin kann die Steuereinheit mit der ersten Abtastschaltung und der mindestens einen weiteren Abtastschaltung gekoppelt sein und eingerichtet sein, die momentan erzeugte Erregerfrequenz an die erste und die mindestens eine weitere Abtastschaltung zu übermitteln. Die erste Abtastschaltung und die mindestens eine weitere Abtastschaltung können dementsprechend die Abtastrate einstellen. Die Steuereinheit kann auch eingerichtet sein, die momentan erzeugte Erregerfrequenz an die Datenverarbeitungseinrichtung zu übermitteln.

Die gegenwärtige Erfindung umfasst weiterhin einen Sensor zum Bestimmen eines die Impedanz einer Suspension anzeigenden Werts, aufweisend: eine Oszillatorschaltung; ein Paar von Erregerelektroden, die mit der Oszillatorschaltung gekoppelt sind, wobei mittels der Oszillatorschaltung über das Paar von Erregerelektroden eine mit einer Erregerfrequenz oszillierende. an der Suspension anliegende Erregerspannung erzeugbar ist; mindestens drei Messelektroden zum Messen eines ersten Stroms in der Suspension zwischen einem ersten Paar der mindestens drei Messelektroden und eines zweiten Stroms in der Suspension zwischen einem zweiten Paar der mindestens drei Messelektroden; und eine Datenverarbeitungseinrichtung, die konfiguriert ist, einen ersten Impedanz-Messwert auf Basis der Erregerspannung und des ersten Stroms zu bestimmen, einen zweiten Impedanz-Messwert auf Basis der Erregerspannung und des zweiten Stroms zu bestimmen, und den die Impedanz der Suspension anzeigenden Wert durch In-Beziehung-Setzen des ersten Impedanz-Messwerts und des zweiten Impedanz-Messwerts zu bestimmen. Die zusätzlichen Merkmale, Modifikationen und technischen Effekte, die oben mit Bezug auf den Sensor beschrieben worden sind, mittels dessen Erregerelektroden ein Erregerstrom erzeugbar ist, gelten für den Sensor, mittels dessen Erregerelektroden eine Erregerspannung erzeugbar ist, analog und sind hiermit explizit für diese alternative Lösung offenbart. Weiterhin sind die obigen Überlegungen für das Verfahren unter Verwendung einer Erregerspannung analog auf den Sensor, mittels dessen Erregerelektroden eine Erregerspannung erzeugbar ist, anwendbar.

Beispielhafte Ausführungsformen der Erfindung umfassen weiterhin ein Computerprogramm bzw. ein Computerprogrammprodukt, welches Programmanweisungen enthält, die bei Ausführung auf einer Datenverarbeitungsanlage ein Verfahren gemäß einer der oben beschriebenen Ausführungsformen durchführen. Dabei können die einzelnen Schritte des Verfahrens durch die Programmanweisungen veranlasst werden und durch andere Komponenten ausgeführt werden oder in der Datenverarbeitungsanlage selbst ausgeführt werden.

Weitere beispielhafte Ausführungsformen der Erfindung werden unten mit Bezug auf die beiliegenden Figuren beschrieben.

Fig. 1 zeigt einen Sensor zum Bestimmen eines die Impedanz einer Suspension anzeigenden Werts gemäß einer beispielhaften Ausführungsform der Erfindung in einer Seitenansicht;

Fig. 2 zeigt einen Sensor gemäß einer beispielhaften Ausführungsform der Erfindung, teilweise dargestellt als Blockschaltbild und teilweise dargestellt als Schaltungsdiagramm;

Fig. 3 zeigt einen gegenüber Fig. 2 modifizierten Sensor gemäß einer weiteren beispielhaften Ausführungsform der Erfindung, teilweise dargestellt als Blockschaltbild und teilweise dargestellt als Schaltungsdiagramm;

Fig. 4 zeigt einen Sensor zum Bestimmen eines die Impedanz einer Suspension anzeigenden Werts gemäß einer weiteren beispielhaften Ausführungsform der Erfindung in einer perspektivischen Ansicht;

Fig. 5 zeigt einen Sensor zum Bestimmen eines die Impedanz einer Suspension anzeigenden Werts gemäß einer weiteren beispielhaften Ausführungsform der Erfindung in einer Seitenansicht;

Fig. 6 zeigt einen Sensor gemäß einer beispielhaften Ausführungsform der Erfindung, teilweise dargestellt als Blockschaltbild und teilweise dargestellt als Schaltungsdiagramm;

Fig. 7 zeigt einen gegenüber Fig. 6 modifizierten Sensor gemäß einer weiteren beispielhaften Ausführungsform der Erfindung, teilweise dargestellt als Blockschaltbild und teilweise dargestellt als Schaltungsdiagramm;

Fig. 8 zeigt einen Sensor zum Bestimmen eines die Impedanz einer Suspension anzeigenden Werts gemäß einer weiteren beispielhaften Ausführungsform der Erfindung in einer Seitenansicht; und

Fig. 9 zeigt einen beispielhaften Verlauf von Permittivitätswerten gegenüber der Erregerfrequenz und illustriert das Herleiten von charakteristischen Eigenschaften der Suspension.

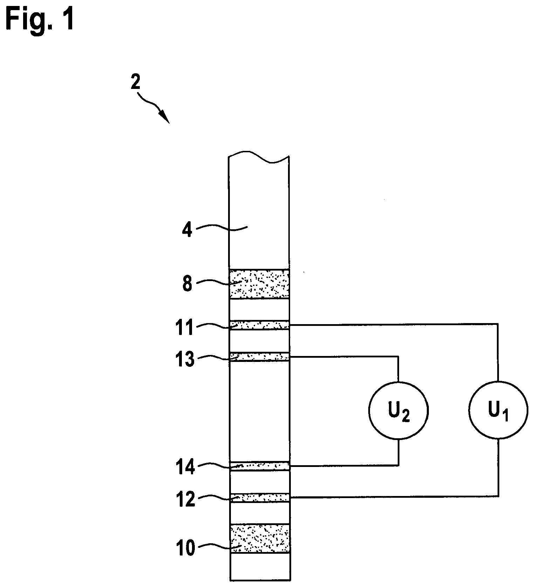

Fig. 1 zeigt einen Sensor 2 gemäß einer beispielhaften Ausführungsform der Erfindung in einer Seitenansicht. Der Sensor 2 ist zum Bestimmen eines die Impedanz einer Suspension anzeigenden Werts ausgebildet. Der Sensor 2 ist zum Eintauchen in die zu analysierende Suspension, insbesondere zum Eintauchen in eine zu analysierende Zellpopulation, ausgebildet. Zu diesem Zweck hat der Sensor 2 einen Stab-förmigen Sensorkörper 4, der in Fig. 1 abgeschnitten gezeigt ist. Der Stab-förmige Sensorkörper 4 kann auch als im Wesentlichen zylindrisch beschrieben werden. Der Stab-förmige Sensorkörper 4 kann eine geeignete Länge haben, so dass die Analyse der Suspension an einer gewünschten Stelle in einem Behälter bzw. Reaktor stattfinden kann, welcher die Suspension beinhaltet.

Der Stab-förmige Sensorkörper 4 hat sechs Elektroden. Insbesondere hat der Stab-förmige Sensorkörper 4 ein Paar von Erregerelektroden, nämlich eine erste Erregerelektrode 8 und eine zweite Erregerelektrode 10, ein erstes Paar von Messelektroden, nämlich eine erste Messelektrode 11 und eine zweite Messelektrode 12, und ein zweites Paar von Messelektroden, nämlich eine dritte Messelektrode 13 und eine vierte Messelektrode 14. In der beispielhaften Ausführungsform von Fig. 1 sind die sechs Elektroden 8, 10, 11, 12, 13 und 14 ringförmig ausgebildet, d.h. sie sind umlaufend um den Stab-förmigen Sensorkörper 4 ausgebildet. Es wird betont, dass die sechs Elektroden auch in anderen geometrischen Konfigurationen vorliegen können, z.B. in länglicher Form entlang des Stab-förmigen Sensorkörpers 4.

In der beispielhaften Ausführungsform von Fig. 1 sind die sechs Elektroden 8, 10, 11, 12, 13 und 14 in einem Endbereich des Stab-förmigen Sensorkörpers 4 angeordnet. Sie können aber auch in jedem anderen geeigneten Bereich des Stab-förmigen Sensorkörpers 4 bzw. des Sensors 2 angeordnet sein.

In der beispielhaften Ausführungsform von Fig. 1 sind das erste Paar von Messelektroden 11, 12 und das zweite Paar von Messelektroden 13, 14 zwischen den Erregerelektroden 8, 10 angeordnet. Insbesondere ist die erste Messelektrode 11 benachbart der ersten Erregerelektrode 8 angeordnet und die dritte Messelektrode 13 benachbart der ersten Messelektrode 11 angeordnet. Weiter insbesondere ist die zweite Messelektrode 12 benachbart der zweiten Erregerelektrode 10 angeordnet und die vierte Messelektrode 14 benachbart der zweiten Messelektrode 12 angeordnet. Das zweite Paar von Messelektroden 13, 14 ist zwischen dem ersten Paar von Messelektroden 11, 12 angeordnet. Die erste Messelektrode 11 und die dritte Messelektrode 13 sind der ersten Erregerelektrode 8 näher als eine gedachte Mittellinie zwischen der ersten Erregerelektrode 8 und der zweiten Erregerelektrode 10. Die zweite Messelektrode 12 und die vierte Messelektrode 14 sind der zweiten Erregerelektrode 10 näher als eine gedachte Mittellinie zwischen der ersten Erregerelektrode 8 und der zweiten Erregerelektrode 10. Durch die Anordnung der Messelektroden 11, 12, 13, 14 zwischen den Erregerelektroden 8, 10 und in der Nähe der Erregerelektroden 8, 10 kann eine vergleichsweise hohe Spannung bei Anliegen des Erregerstroms gemessen werden.

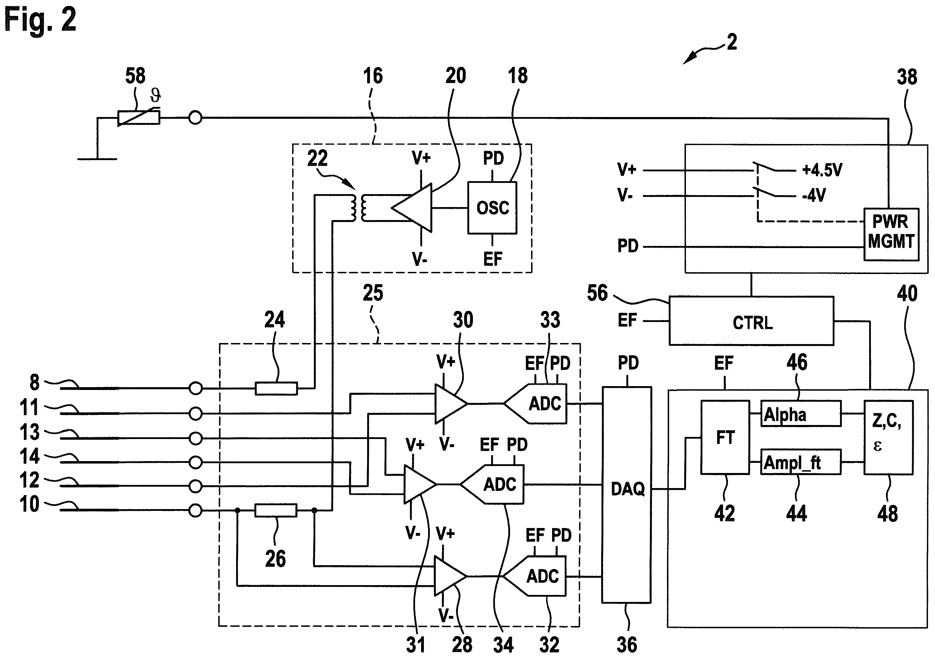

Im Betrieb werden eine erste Spannung $U_1$ zwischen dem ersten Paar von Messelektroden 11, 12 und eine zweite Spannung $U_2$ zwischen dem zweiten Paar von Messelektroden 13, 14 gemessen. Dies ist in Fig. 1 schematisch angedeutet und wird im Detail unten mit Bezug auf Fig. 2 beschrieben.

Fig. 2 zeigt einen Sensor 2 gemäß einer beispielhaften Ausführungsform der Erfindung, teilweise in einem Blockdiagramm und teilweise in einem Schaltungsdiagramm dargestellt. Die Komponenten des Sensors 2 der Fig. 2 können in einem Sensor der in Fig. 1 gezeigten körperlichen Gestalt vorhanden sein. Das heißt, bei dem schaltungstechnischen bzw. signalverarbeitungstechnischen Aufbau des Sensors 2 der Fig. 2 kann es sich um den Aufbau der elektrischen Komponenten des Sensors 2 der Fig. 1 handeln. Dabei können die Komponenten, die in Fig. 2 rechts von den kreisförmig gezeichneten Kopplungspunkten gezeigt sind, in dem Sensorkörper 4 oder in einem darin anschließenden Bauteil untergebracht sein.

Der Sensor 2 hat die oben beschriebene erste Erregerelektrode 8, zweite Erregerelektrode 10, erste Messelektrode 11, zweite Messelektrode 12, dritte Messelektrode 13 und vierte Messelektrode 14. Die sechs Elektroden 8, 10, 11, 12, 13, 14 sind von außen zugänglich, d.h. sie stehen mit der Suspension in Kontakt, wenn der Sensor 2 zur Analyse der Suspension in die Suspension eingetaucht ist. Weiterhin ist ein Temperatursensor 58 vorgesehen, der außerhalb des Gehäuses des Sensors 2 liegt.

Der Sensor 2 weist eine Oszillatorschaltung 16, eine Signal-Erfassungs- und Aufbereitungs-Schaltung 25, einen Datenspeicher 36, eine Datenverarbeitungseinrichtung 40, eine Steuereinheit 56 und eine Power-Management-Einheit 38 auf. Die einzelnen Komponenten und die Funktionsweise dieser Teilsysteme werden im Folgenden detailliert beschreiben.

Die Oszillatorschaltung 16 weist einen Oszillator 18 auf, der mit einem Oszillationsverstärker 20 gekoppelt ist, welcher wiederum mit einem Transformator 22 gekoppelt ist. Der Oszillator 18 wird über einen Steuereingang mit der gewünschten Erregerfrequenz EF versorgt. Die Erregerfrequenz EF wird von der Steuereinheit 56 festgelegt, wie unten im Detail beschrieben, und an den Oszillator 18 übergeben. Der Oszillator 18 erzeugt eine Schwingung mit der Erregerfrequenz EF, welche an den Oszillationsverstärker 20 übergeben wird. Der Oszillationsverstärker 20 erzeugt einen Erregerstrom mit der Erregerfrequenz EF durch die Primärwicklung des Transformators 22. Per Induktion wird der Erregerstrom auf die Sekundärwicklung des Transformators 22 übertragen, von wo aus der Strom an die erste und die zweite Erregerelektrode 8, 10 angelegt wird. Dabei ist das eine Ende der Sekundärwicklung über einen ersten Widerstand 24 mit der ersten Erregerelektrode 8 verbunden und das zweite Ende der Sekundärwicklung über einen zweiten Widerstand 26 mit der zweiten Erregerelektrode 10 verbunden. Somit ergibt sich ein geschlossener Stromkreis von dem ersten Ende der Sekundärwicklung durch den ersten Widerstand 24, über die erste Erregerelektrode 8 durch die Suspension zu der zweiten Erregerelektrode 10, und durch den zweiten Widerstand 26 zu dem zweiten Ende der Sekundärwicklung. Auf diese Weise wird ein mit der Erregerfrequenz EF oszillierender Erregerstrom durch die Suspension zwischen der ersten Erregerelektrode 8 und der zweiten Erregerelektrode 10 erzeugt. In der beispielhaften Ausführungsform der Fig. 2 ist der Erregerstrom ein sinusförmiger, mit der Erregerfrequenz EF oszillierender Erregerstrom. Weiterhin hat der Erregerstrom in der beispielhafter Ausführungsform der Fig. 2 eine Amplitude von 1 Vpp bis 2 Vpp.

Der Transformator 22 sorgt für eine galvanische Entkopplung zwischen dem Oszillationsverstärker 20 und der ersten und zweiten Erregerelektrode 8, 10. Es kann eine Kopplungskapazität parallel zu dem Transformator 22 vorgesehen sein. Man kann dann auch davon sprechen, dass der Transformator 22 eine parallele Kapazität hat, welche zwischen der Primärwicklung und der Sekundärwicklung vorhanden ist. Die parallele Kapazität kann ein diskretes Bauteil oder eine parasitäre Kapazität des Transformators sein. Die parallele Kapazität kann durch ihre Anordnung parallel zu dem Transformator störenden Einflüssen von anderen Kopplungskapazitäten, wie beispielsweise Kopplungskapazitäten zwischen den Elektroden und dem Behälter der Suspension und/oder Kopplungskapazitäten zwischen den Elektroden und anderen in der Suspension vorhandenen Sensoren, entgegenwirken. Die parallele Kapazität kann zwischen 1 pF und 5 pF betragen.

Durch den mit der Erregerfrequenz EF oszillierenden Erregerstrom zwischen der ersten Erregerelektrode 8 und der zweiten Erregerelektrode 10 entsteht eine erste Wechselspannung zwischen der ersten Messelektrode 11 und der zweiten Messelektrode 12 sowie eine zweite Wechselspannung zwischen der dritten Messelektrode 13 und der vierten Messelektrode 14. Sowohl der Erregerstrom als auch die erste Spannung zwischen dem ersten Paar von Messelektroden 11, 12 als auch die zweite Spannung zwischen dem zweiten Paar von Messelektroden 13, 14 werden durch die Signal-Erfassungs- und Aufbereitungs-Schaltung 25 erfasst und abgetastet. Am Ende der Signalverarbeitung in der Signal-Erfassungs- und Aufbereitungs-Schaltung 25 stehen Digitalsignale für den Erregerstrom, die erste Spannung und die zweite Spannung.

Ein erstes Signal, welches den Erregerstrom abbildet, wird auf folgende Weise gewonnen. Der zweite Widerstand 26 fungiert als Messwiderstand für den Erregerstrom. Die Spannung über den Messwiderstand 26 wird mittels zweier Leiter abgegriffen und als das erste Signal einer ersten Verstärkerschaltung 28 zugeführt. Das verstärkte erste Signal wird dem ersten Analog-Digital-Wandler 32 zugeführt. Dort wird das verstärkte erste Signal in ein digitales Signal umgewandelt d.h. das verstärkte erste Signal wird abgetastet und quantisiert. Die resultierenden ersten Abtastwerte werden an den Datenspeicher 36 ausgegeben. Es ist ersichtlich, dass der erste Widerstand 24 nicht für die Gewinnung des ersten Signals erforderlich ist. Aus Symmetriegründen ist der erste Widerstand 24 jedoch trotzdem vorgesehen. Weiterhin ist ersichtlich, dass das Abgreifen eines den Erregerstrom abbildenden Signals auch an dem ersten Widerstand 24 stattfinden kann. Das heißt, die erste Verstärkerschaltung könnte auch mit der ersten Erregerelektrode 8 bzw. dem ersten Widerstand 24 gekoppelt sein. Der erste Widerstand 24 und der zweite Widerstand 26 können beispielsweise jeweils einen Wert von 30 $\Omega$ bis 50 $\Omega$ haben.

Die Spannung zwischen der ersten Messelektrode 11 und der zweiten Messelektrode 12 bildet ein zweites Signal, welches einer zweiten Verstärkerschaltung 30 zugeführt wird. Dort wird das zweite Signal verstärkt, und das verstärkte zweite Signal wird einem zweiten Analog-Digital-Wandler 33 zugeführt. Der zweite Analog-Digital-Wandler 33 erzeugt, analog zu dem ersten Analog-Digital-Wandler 32, zweite Abtastwerte, welche zeitdiskret und quantisiert sind. Die zweiten Abtastwerte werden ebenfalls an den Datenspeicher 36 ausgegeben.

Die Spannung zwischen der dritten Messelektrode 13 und der vierten Messelektrode 14 bildet ein drittes Signal, welches einer dritten Verstärkerschaltung 31 zugeführt wird. Dort wird das dritte Signal verstärkt, und das verstärkte dritte Signal wird einem dritten Analog-Digital-Wandler 34 zugeführt. Der dritte Analog-Digital-Wandler 34 erzeugt, analog zu dem ersten Analog-Digital-Wandler 32, dritte Abtastwerte, welche zeitdiskret und quantisiert sind. Die dritten Abtastwerte werden ebenfalls an den Datenspeicher 36 ausgegeben.

Der erste Analog-Digital-Wandler 32, der zweite Analog-Digital-Wandler 33 und der dritte Analog-Digital-Wandler 34 erhalten ebenfalls die Information über die Erregerfrequenz EF von der Steuereinheit 56. Der erste Analog-Digital-Wandler 32, der zweite Analog-Digital-Wandler 33 und der dritte Analog-Digital-Wandler 34 benutzen das 4-fache der Erregerfrequenz EF für das Abtasten des verstärkten ersten Signals, des verstärkten zweiten Signals und des verstärkten dritten Signals. Somit erstellen der erste Analog-Digital-Wandler 32, der zweite Analog-Digital-Wandler 33 und der dritte Analog-Digital-Wandler 34 erste, zweite und dritte Abtastwerte für den Erregerstrom, die erste Spannung und die zweite Spannung mit dem 4-fachen der Erregerfrequenz EF.

Die von dem ersten Analog-Digital-Wandler 32, dem zweiten Analog-Digital-Wandler 33 und dem dritten Analog-Digital-Wandler 34 ausgegebenen ersten, zweiten und dritten Abtastwerte werden in dem Datenspeicher 36 zwischengespeichert. Der Datenspeicher 36 stellt somit ein Depot dar, das die ersten Abtastwerte, die zweiten Abtastwerte und die dritten Abtastwerte aufnimmt und von der Echtzeit unabhängig für die weitere Datenverarbeitung zur Verfügung stellen kann. Somit gibt es ab dem Datenspeicher 36 keine Echtzeitanforderungen an die nachgelagerten Bauelemente mehr. Im Gegenteil, die nachgelagerten Bauelemente können auf eine über einen gewissen Zeitraum angesammelte Datenbasis in dem Datenspeicher 36 zugreifen. Der Datenspeicher 36 kann beispielsweise ein DPRAM oder jede andere geeignete Art von Datenspeicher sein.

Der Datenspeicher 36 ist mit der Datenverarbeitungseinrichtung 40 gekoppelt und gibt die ersten Abtastwerte für den Erregerstrom, die zweiten Abtastwerte für die erste Spannung zwischen der ersten Messelektrode 11 und der zweiten Messelektrode 12 und die dritten Abtastwerte für die zweite Spannung zwischen der dritten Messelektrode 13 und der vierten Messelektrode 14 an die Datenverarbeitungseinrichtung 40 aus.

In der Datenverarbeitungseinrichtung 40 werden die ersten, zweiten und dritten Abtastwerte an ein Fourier-Transformations-Modul 42 übergeben. Das Fourier-Transformations-Modul 42 führt zwei diskrete, komplexe Fourier-Transformationen mit den Abtastwerten durch. Insbesondere führt das Fourier-Transformations-Modul 42 eine erste diskrete, komplexe Fourier-Transformation mit den ersten Abtastwerten für den Erregerstrom und den zweiten Abtastwerten für die Spannung zwischen der ersten Messelektrode 11 und der zweiten Messelektrode 12, d.h. mit den Abtastwerten für den Erregerstrom und den Abtastwerten für die erste Spannung, durch. Weiter insbesondere führt das Fourier-Transformations-Modul 42 eine zweite diskrete, komplexe Fourier-Transformation mit den ersten Abtastwerten für den Erregerstrom und den dritten Abtastwerten für die Spannung zwischen der dritten Messelektrode 13 und der vierten Messelektrode 14, d.h. mit den Abtastwerten für den Erregerstrom und den Abtastwerten für die zweite Spannung, durch.

Die in dem Fourier-Transformations-Modul 42 durchgeführten Fourier-Transformationen sind diskret und komplex, weil die zeitdiskreten Abtastwerte für Erregerstrom und für die jeweilige Spannung als voneinander abhängige Größen analysiert werden. Das Ergebnis dieser komplexen Fourier-Transformationen sind die Amplituden von Erregerstrom und jeweiliger gemessener Spannung für verschiedene Frequenzen sowie die Phasenverschiebung $\alpha$ zwischen Erregerstrom und jeweiliger gemessener Spannung für die verschiedenen Frequenzen. Dabei ist es möglich, dass die Fourier-Transformationen eine breite spektrale Analyse der Abtastwerte vornehmen und dann alle Spektralkomponenten mit Ausnahme der Spektralkomponenten bei der Erregerfrequenz EF verworfen werden. Es ist aber auch möglich, dass die Fourier-Transformationen gezielt die Spektralkomponente des Erregerstroms sowie die Spektralkomponente der jeweiligen Spannung zwischen den jeweiligen Messelektroden bei der Erregerfrequenz bestimmt. In diesem Zusammenhang kann auch der Goertzel-Algorithmus zum Einsatz kommen, mit dem gezielt die spektralen Komponenten bei der Erregerfrequenz EF bestimmt werden können.

Die Amplitude der Spektralkomponente des Erregerstroms bei der Erregerfrequenz EF und die Amplitude der Spektralkomponente der jeweiligen gemessenen Spannung bei der Erregerfrequenz EF werden über eine erste Datenübertragungsverbindung 44 an ein Impedanz- und Permittivität-Bestimmungs-Modul 48 übergeben. Die Phasenverschiebung $\alpha$ zwischen der Spektralkomponente des Erregerstroms bei der Erregerfrequenz und der Spektralkomponente der jeweiligen gemessenen Spannung bei der Erregerfrequenz wird über eine zweite Datenübertragungsverbindung 46 an das Impedanz- und Permittivität-Bestimmungs-Modul 48 übergeben.

Das Impedanz- und Permittivität-Bestimmungs-Modul 48 bestimmt aus den übergebenen Parametern einen ersten Impedanz-Messwert $Z_{sig}|_1$, einen zweiten Impedanz-Messwert $Z_{sig}|_2$, einen Impedanz-Wert Z, einen Kapazitätswert C und die Permittivität $\varepsilon$ der Suspension bei der Erregerfrequenz. Der erste Impedanz-Messwert $Z_{sig}|_1$ ergibt sich aus der Amplitude des Erregerstroms bei der Erregerfrequenz EF, der Amplitude der ersten Spannung bei der Erregerfrequenz EF und der Phasenverschiebung $\alpha$ zwischen Erregerstrom und erster Spannung bei der Erregerfrequenz EF. Der erste Impedanz-Messwert $Z_{sig}|_1$ ist somit ein komplexer Impedanz-Messwert bei der Erregerfrequenz EF. Der zweite Impedanz-Messwert $Z_{sig}|_2$ ergibt sich aus der Amplitude des Erregerstroms bei der Erregerfrequenz EF, der Amplitude der zweiten Spannung bei der Erregerfrequenz EF und der Phasenverschiebung $\alpha$ zwischen Erregerstrom und zweiter Spannung bei der Erregerfrequenz EF. Der zweite Impedanz-Messwert $Z_{sig}|_2$ ist somit ein komplexer Impedanz-Messwert bei der Erregerfrequenz EF. Wie oben beschrieben, liegen die genannten Amplituden von Erregerstrom, erster Spannung und zweiter Spannung sowie die genannten Phasenverschiebungen als Ergebnisse der ersten und zweiten komplexen Fourier-Transformation vor. Somit können der erste Impedanz-Messwert $Z_{sig}|_1$ und der zweite Impedanz-Messwert $Z_{sig}|_2$ bequem aus den in dem Impedanz- und Permittivität-Bestimmungs-Modul 48 vorhandenen Daten berechnet werden. Es wird betont, dass der erste Impedanz-Messwert $Z_{sig}|_1$ und der zweite Impedanz-Messwert $Z_{sig}|_2$ auch auf andere Art und Weise aus dem ersten Signal, d.h. der an dem zweiten Widerstand 26 abgegriffenen Spannung, dem zweiten Signal, d.h. der an dem ersten Paar von Messelektroden 11, 12 abgegriffenen Spannung, und dem dritten Signal, d.h. der an dem zweiten Paar von Messelektroden 13, 14 abgegriffenen Spannung, bestimmt werden können. Die oben im Detail beschriebene Signalverarbeitung erlaubt zwar ein besonders genaues Bestimmen des ersten Impedanz-Messwerts $Z_{sig}|_1$ und des zweiten Impedanz-Messwerts $Z_{sig}|_2$. Aber für das im Folgenden beschrieben Bestimmen des Impedanz-Werts Z ist die Art und Weise der Bestimmung des ersten Impedanz-Messwerts $Z_{sig}|_1$ und des zweiten Impedanz-Messwerts $Z_{sig}|_2$ nicht ausschlaggebend. Insofern kann jede geeignete Signalverarbeitung verwendet werden.

Das Impedanz- und Permittivität-Bestimmungs-Modul 48 bestimmt den Impedanz-Wert Z gemäß der folgenden Formel:

$$Z = k \frac{1}{(\lambda_1 - \lambda_2)} \left( G_{el}^{-1}(Z_{sig})\big|_1 - G_{el}^{-1}(Z_{sig})\big|_2 \right).$$

Dabei bezeichnet $Z_{sig}|_1$ den ersten Impedanz-Messwert und $Z_{sig}|_2$ den zweiten Impedanz-Messwert, wie oben beschrieben. $G_{el}^{-1}$ bezeichnet eine Korrekturfunktion, die das Übertragungsverhalten der Messanordnung abbildet. In der beispielhaften Ausführungsform von Fig. 2 korrigiert $G_{el}^{-1}$ diejenigen Artefakte, die zwischen den jeweiligen Elektroden und den zugehörigen Analog-Digital-Wandlern in die Signale eingebracht worden sind. Dazu können beispielsweise Laufzeitunterschiede in den einzelnen Signalwegen, nicht lineare Verstärkungen in den Verstärkerschaltungen, etc. gehören. Demnach stellt $G_{el}^{-1}$ diejenigen Impedanz-Messwerte ganz oder zumindest näherungsweise wieder her, die an den Elektroden direkt angelegen haben. Weiterhin bezeichnet $\lambda_1$ einen ersten Geometriefaktor, der die Messgeometrie des ersten Paars von Messelektroden 11, 12 abbildet, und $\lambda_2$ einen zweiten Geometriefaktor, der die Messgeometrie des zweiten Paars von Messelektroden 13, 14 abbildet. Der erste Geometriefaktor $\lambda_1$ und der zweite Geometriefaktor $\lambda_2$ beschreiben die jeweiligen Messzellen, die den Spannungsmessungen an dem ersten Paar von Messelektroden 11, 12 und an dem zweiten Paar von Messelektroden 13, 14 zugrunde liegen. Die Natur des ersten Geometriefaktors $\lambda_1$ und des zweiten Geometriefaktors $\lambda_2$ wird weiter unten im Detail beschrieben. Die Variable k bezeichnet eine Proportionalitätskonstante.

Durch die Verwendung des ersten Impedanz-Messwerts $Z_{sig}|_1$ und des zweiten Impedanz-Messwerts $Z_{sig}|_2$ und das In-Beziehung-Setzen des ersten Impedanz-Messwerts $Z_{sig}|_1$ und des zweiten Impedanz-Messwerts $Z_{sig}|_2$ gemäß obiger Formel kann ein Impedanz-Wert Z bestimmt werden, der sehr robust gegen Störeinflüsse ist. Insbesondere kann durch die Differenzbildung der mit der Korrekturfunktion $G_{el}^{-1}$ angepassten Impedanz-Messwerte und die Differenzbildung der Geometriefaktoren erreicht werden, dass sich Störeinflüsse niedriger Ordnung in den zwei Messungen gegenseitig aufheben. Der durch die obige Formel erhaltene Impedanz-Wert Z enthält nur noch Störeinflüsse höherer Ordnung, die in vielen Anwendungsfällen vergleichsweise klein sind. Somit kann eine hohe Messgenauigkeit erreicht werden.

Der Kapazitätswert C und die Permittivität ε werden im Anschluss aus dem Impedanz-Wert Z berechnet. Auf diese Weise wird im Ergebnis aus dem Impedanz-Wert Z die Materialeigenschaft Permittivität ε der Suspension hergeleitet. Für die Herleitung der Permittivität ε können an sich bekannte Ansätze und Verfahren verwendet werden. Durch die hochgenaue Gewinnung des Impedanz-Werts Z, wie hierin beschrieben, können auch bei Verwendung von bekannten Verfahren zur Herleitung der Permittivität ε verbesserte Ergebnisse im Vergleich zu früheren Sensoren erzielt werden.

Somit stehen der Impedanz-Wert Z, der Kapazitätswert C und die Permittivität ε als Ergebnisse der Signalverarbeitung in der Datenverarbeitungseinrichtung 40 zur Verfügung. Insbesondere stehen diese Werte als Ergebnisse für die Erregung der Suspension mit einer bestimmten Erregerfrequenz zur Verfügung. Einer oder mehrere dieser Werte können zur Weiterverarbeitung ausgegeben werden. Die Ausgabe kann an eine externe Einheit oder, wie in der beispielhaften Ausführungsform der Fig. 2 dargestellt, an die in dem Sensor 2 vorhandene Steuereinheit 56 erfolgen. In der beispielhaften Ausführungsform der Fig. 2 wird der für die Permittivität ε bestimmte Wert an die Steuereinheit 56 ausgegeben.

Die Datenverarbeitungseinrichtung 40 kann in Software implementiert vorliegen oder als Anordnung von Hardware-Komponenten vorliegen. Es ist auch möglich, dass die Datenverarbeitungseinrichtung 40 teilweise in Software und teilweise in Hardware implementiert ist. Gleiches gilt für die unten beschriebene Steuereinheit 56.

Die Steuereinheit 56 ist mit der Power-Management-Einheit 38, mit der Oszillatorschaltung 16, mit der Signal-Erfassungs- und Aufbereitungs-Schaltung 25 und mit der Datenverarbeitungseinrichtung 40 verbunden. Die Steuereinheit 56 steuert das Verfahren zum Bestimmen eines die Impedanz einer Suspension anzeigenden Werts gemäß beispielhaften Ausführungsformen der Erfindung. Dazu ist die Steuereinheit 56 eingerichtet, die Erregerfrequenz EF für das Verfahren festzulegen. Insbesondere ist die Steuereinheit eingerichtet, nacheinander eine Mehrzahl von Erregerfrequenzen für eine Mehrzahl von Durchläufen des Verfahrens im Rahmen einer Impedanzspektroskopie festzulegen.

Für einen gegebenen Durchlauf übermittelt die Steuereinheit 56 die festgelegte Erregerfrequenz EF an die Oszillatorschaltung 16, wo der Oszillator 18 eine Schwingung mit der Erregerfrequenz EF erzeugt, an die Signal-Erfassungs- und Aufbereitungs-Schaltung 25, wo der erste Analog-Digital-Wandler 32, der zweite Analog-Digital-Wandler 33 und der dritte Analog-Digital-Wandler 34 die Abtastrate auf Basis der Erregerfrequenz EF einstellen, und an die Datenverarbeitungseinrichtung 40, wo das Fourier-Transformations-Modul 42 die Abtastwerte von Erregerstrom, erster Spannung und zweiter Spannung bezüglich der spektralen Signalkomponente bei der Erregerfrequenz analysiert.

Weiterhin ist die Steuereinheit 56 in der beispielhaften Ausführungsform von Fig. 2 mit der Datenverarbeitungseinrichtung 40 insofern gekoppelt, dass die Datenverarbeitungseinrichtung 40 die für die Erregerfrequenz EF bestimmte Permittivität ε an die Steuereinheit 56 übermittelt. Die Steuereinheit 56 kann dann im Rahmen einer Impedanzspektroskopie eine neue Erregerfrequenz für den nächsten Durchlauf des Verfahrens festlegen.

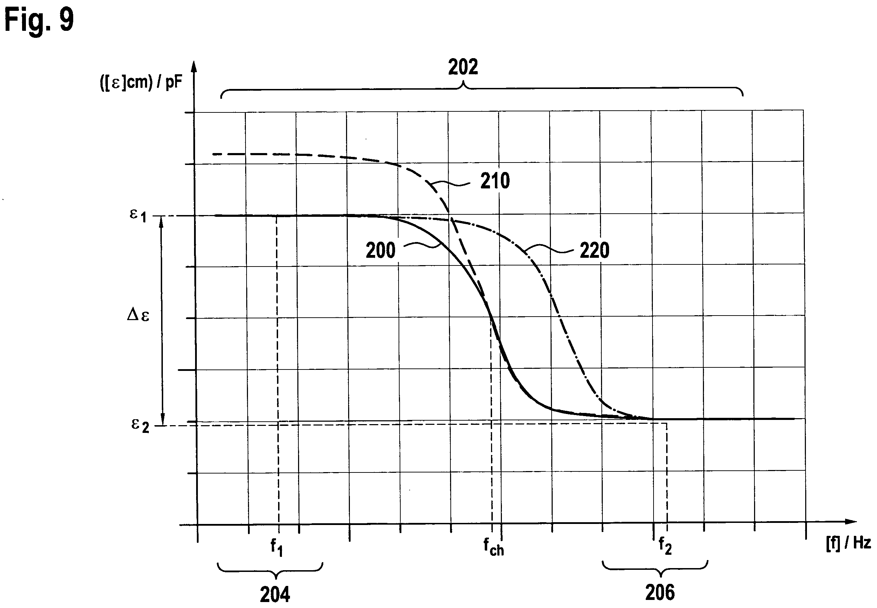

Nachdem eine Mehrzahl von Permittivitätswerten für verschiedene Erregerfrequenzen bestimmt worden ist, kann die Steuereinheit 56 eine oder mehrere charakteristische Eigenschaften der Suspension aus der Mehrzahl von Permittivitätswerten herleiten. Dazu kann die Steuereinheit eine Kurve durch die Mehrzahl von Permittivitätswerten legen und aus der Kurve die charakteristischen Eigenschaften der Suspension herleiten, wie unten mit Bezug auf Fig. 9 beschrieben. Ein solches In-Beziehung-Setzen der Mehrzahl von Permittivitätswerten kann auch außerhalb des Sensors 2 erfolgen.

Die Steuereinheit 56 ist mit der Power-Management-Schaltung 38 gekoppelt, um den Beginn und das Ende eines Durchlaufs des Verfahrens zu signalisieren. Die Power-Management-Schaltung 38 versorgt auf der Grundlage dieser Signale den Oszillationsverstärker 20 sowie die erste Verstärkerschaltung 28, die zweite Verstärkerschaltung 30 und die dritte Verstärkerschaltung 31 mit der positiven Versorgungsspannung V+ und der negative Versorgungsspannung V-, welche in der vorliegenden beispielhaften Ausführungsform +4,5 V und -4 V sind. Nach dem Ende eines Durchlaufs des Verfahrens trennt die Power-Management-Schaltung 38 die positive und die negative Versorgungsspannung und übermittelt an den Oszillator 18, den ersten Analog-Digital-Wandler 32, den zweiten Analog-Digital-Wandler 33, den dritten Analog-Digital-Wandler 34 und den Datenspeicher 36 ein Abschaltsignal ("Power Down"). Auf diese Weise kann der Sensor zwischen den Durchläufen des Verfahrens zum Bestimmen des die Impedanz anzeigenden Werts elektrische Energie sparen.

Die Power-Management-Schaltung 38 kann die elektrische Energie von außerhalb des Sensors 2 beziehen oder über ein internes Energie-Reservoir, z.B. in Form einer Batterie, verfügen.

Zum Schutz des Sensors 2 kann die Power-Management-Schaltung 38 die Spannungsversorgung öffnen, wenn der Temperatursensor 58 eine Temperatur oberhalb eines vorbestimmten Schwellwerts misst.

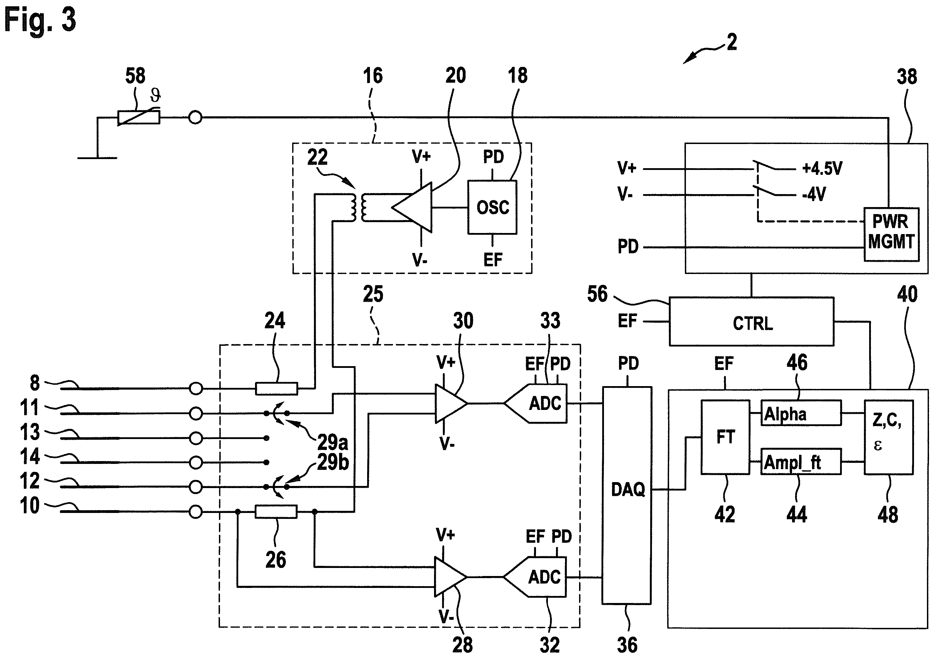

Fig. 3 zeigt einen Sensor 2 gemäß einer gegenüber Fig. 2 modifizierten, weiteren beispielhaften Ausführungsform der Erfindung, wiederum teilweise in einem Blockdiagramm und teilweise in einem Schaltungsdiagramm dargestellt. Insbesondere bezieht sich die Modifikation auf die Signalverarbeitung in der Signal-Erfassungs- und Aufbereitungs-Schaltung 25. Im Allgemeinen sind entsprechende Komponenten mit den gleichen Bezugszeichen wie in Fig. 2 versehen.

Für deren Beschreibung wird auf die obigen Ausführungen Bezug genommen.

Die Signal-Erfassungs- und Aufbereitungs-Schaltung 25 der Ausführungsform der Fig. 3 hat keine dritte Verstärkerschaltung 31 und keinen dritten Digital-AnalogWandler 34. Stattdessen kann die zweite Verstärkerschaltung 30 wahlweise mit dem ersten Paar von Messelektroden 11, 12 oder mit dem zweiten Paar von Messelektroden 13, 14 verbunden werden. Dazu sind ein erster Auswahlschalter 29a und ein zweiter Auswahlschalter 29b vorgesehen. Der erste Auswahlschalter 29a verbindet entweder die erste Messelektrode 11 oder die dritte Messelektrode 13 mit der zweiten Verstärkerschaltung 30. Der zweite Auswahlschalter 29b verbindet entweder die zweite Messelektrode 12 oder die vierte Messelektrode 14 mit der zweiten Verstärkerschaltung 30. Somit kann über die zweite Verstärkerschaltung 30 entweder die erste Spannung, d.h. die Spannung zwischen der ersten und zweiten Messelektrode 11, 12, oder die zweite Spannung, d.h. die Spannung zwischen der dritten und vierten Messelektrode 13, 14, an den zweiten Analog-Digital-Wandler 33 weitergeleitet werden.

Für das Verfahren zum Bestimmen des die Impedanz der Suspension anzeigenden Werts bedeutet die Modifikation der Fig. 3, dass der erste Impedanz-Messwert und der zweite Impedanz-Messwert auf der Basis von Signalen bestimmt werden, die zeitlich versetzt voneinander abgegriffen werden. Jedoch kann das Verfahren mit einem Sensor 2 implementiert werden, der zusätzlich zu der ersten Verstärkerschaltung 28 nur eine weitere Verstärkerschaltung 30 und zusätzlich zu dem ersten Analog-Digital-Wandler 32 nur einen weiteren Analog-Digital-Wandler 33 aufweist.

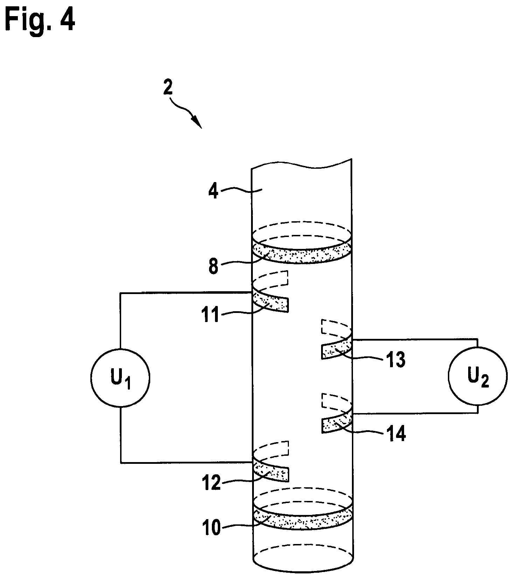

Fig. 4 zeigt einen Sensor 2 gemäß einer gegenüber Fig. 1 modifizierten, weiteren beispielhaften Ausführungsform der Erfindung in einer perspektivischen Ansicht. Die Modifikation bezieht sich auf die geometrische Anordnung des ersten und zweiten Paars von Messelektroden. Im Vergleich zu der Ausführungsform der Fig. 1 sind die erste Messelektrode 11, die zweite Messelektrode 12, die dritte Messelektrode 13 und die vierte Messelektrode 14 nicht ringförmig ausgebildet, son-dern teilringförmig ausgebildet. Jede der vier Messelektroden erstreckt sich über einen Kreissektor von etwas weniger als 180° entlang der zylindrischen Außenfläche des Stab-förmigen Sensorkörpers 4. In der Ansicht von Fig. 4 sind die erste Messelektrode 11 und die zweite Messelektrode 12 auf der linken Seite des Sensorkörpers 4 angeordnet, und die dritte Messelektrode 13 und die vierte Messelektrode 14 sind auf der rechten Seite des Sensorkörpers 4 angeordnet. In anderen Worten, das erste Paar von Messelektroden 11, 12 und das zweite Paar von Messelektroden 13, 14 sind auf unterschiedlichen Seiten des Sensors 2 angeordnet. Eine solche Anordnung ermöglicht einen geringen wechselseitigen Einfluss der Elektrodenpaare aufeinander, welcher sich potentiell negativ auf die Messgenauigkeit auswirken könnte.

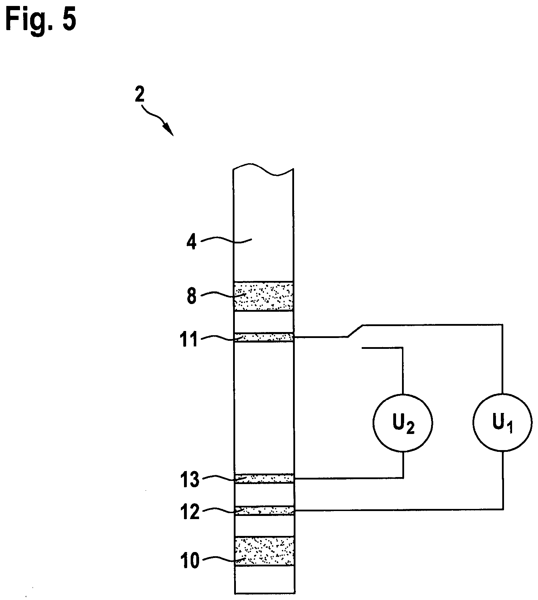

Fig. 5 zeigt einen Sensor 2 gemäß einer gegenüber Fig. 1 modifizierten, weiteren beispielhaften Ausführungsform der Erfindung in einer Seitenansicht. Die Modifikation bezieht sich auf die Anzahl und die geometrische Anordnung des Messelektroden. Der Sensor 2 der beispielhaften Ausführungsform der Fig. 5 hat drei Messelektroden, eine erste Messelektrode 11, eine zweite Messelektrode 12 und eine dritte Messelektrode 13. Die erste Messelektrode 11 und die zweite Messelektrode 12 entsprechen in ihrer Anordnung dem ersten Paar von Messelektroden 11, 12 der Ausführungsform der Fig. 1. Die dritte Messelektrode 13 der Ausführungsform der Fig. 5 ist dort angeordnet, wo in der Ausführungsform der Fig. 1 die vierte Messelektrode 14 angeordnet war.

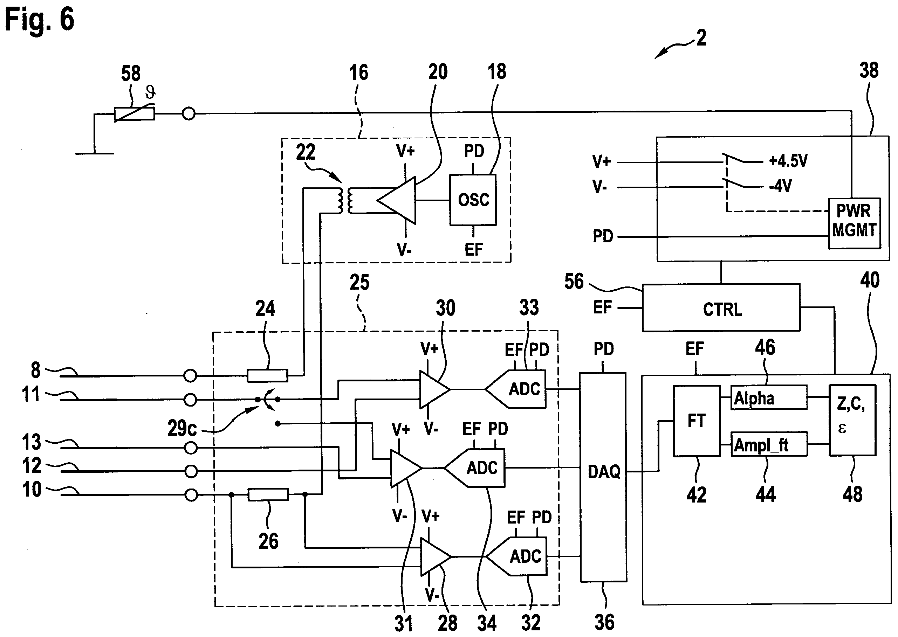

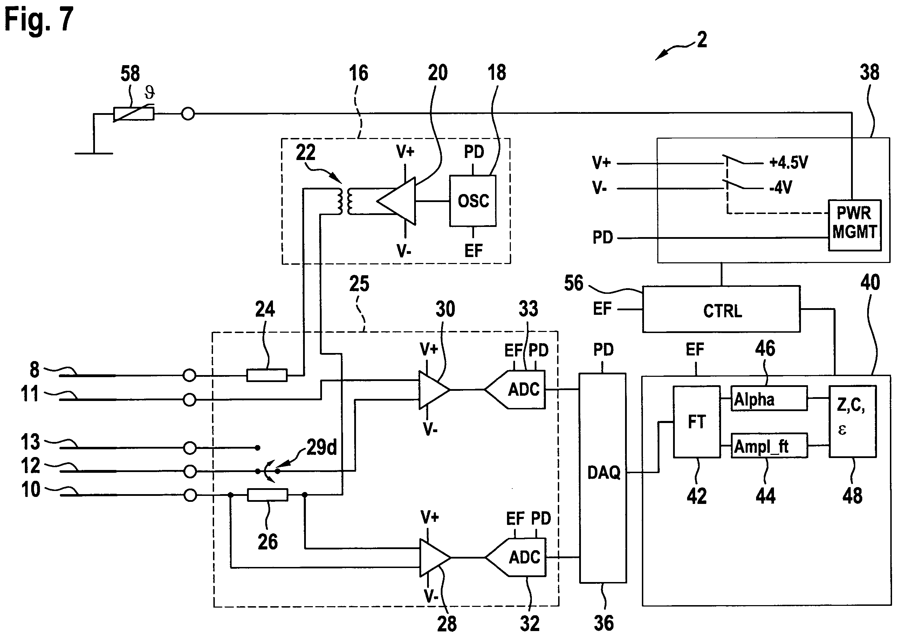

In der beispielhaften Ausführungsform der Fig. 5 besteht das erste Paar von Messelektroden aus der ersten Messelektrode 11 und der zweiten Messelektrode 12. Das zweite Paar von Messelektroden besteht aus der ersten Messelektrode 11 und der dritten Messelektrode 13. Im Betrieb wird eine erste Spannung $U_1$ an dem ersten Paar von Messelektroden 11, 12 gemessen, während eine zweite Spannung $U_2$ an dem zweiten Paar von Messelektroden 11, 13 gemessen wird. In anderen Worten, die erste Messelektrode 11 bildet einen Potentialbezugspunkt sowohl für die Messung der ersten Spannung als auch für die Messung der zweiten Spannung. Zwei beispielhafte Ausführungsformen der nachgelagerten Signalverarbeitung des Sensors 2 der Fig. 5 werden im Folgenden mit Bezug auf die Fig. 6 und 7 beschrieben.

Fig. 6 zeigt einen Sensor 2 gemäß einer weiteren beispielhaften Ausführungsform der Erfindung, teilweise in einem Blockdiagramm und teilweise in einem Schaltungsdiagramm dargestellt. Die Komponenten des Sensors 2 der Fig. 6 können in einem Sensor der in Fig. 5 gezeigten körperlichen Gestalt vorhanden sein. Das heißt, bei dem schaltungstechnischen bzw. signalverarbeitungstechnischen Aufbau des Sensors 2 der Fig. 6 kann es sich um den Aufbau der elektrischen Komponenten des Sensors 2 der Fig. 5 handeln. Somit verhält sich Fig. 6 zu Fig. 5 so wie Fig. 2 zu Fig. 1. Der Sensor 2 der Fig. 6 ist insgesamt sehr ähnlich und in weiten Teilen identisch zu dem Sensor 2 der Fig. 2. Entsprechende Komponenten sind mit entsprechenden Bezugszeichen versehen. Für die Beschreibung der Komponenten wird auf die Beschreibung der Fig. 2 oben Bezug genommen.

Die Änderungen der Ausführungsform der Fig. 6 gegenüber der Ausführungsform der Fig. 2 tragen der Tatsache Rechnung, dass der Sensor 2 der Fig. 6 nur drei Messelektroden 11, 12 und 13 hat, wie oben mit Bezug auf Fig. 5 dargelegt. Die erste Messelektrode 11 kann mittels eines Auswahlschalters 29c selektiv mit der zweiten Verstärkerschaltung 30 oder der dritten Verstärkerschaltung 31 verbunden werden. Die zweite Messelektrode 12 ist mit der zweiten Verstärkerschaltung 30 verbunden, und die dritte Messelektrode 13 ist mit der dritten Verstärkerschaltung 31 verbunden. Somit kann entweder über die zweite Verstärkerschaltung 30 die erste Spannung an den zweiten Analog-Digital-Wandler 33 weitergegeben werden oder über die dritte Verstärkerschaltung 31 die zweite Spannung an den dritten Analog-Digital-Wandler 34 weitergegeben werden. Für das Verfahren zum Bestimmen des die Impedanz der Suspension anzeigenden Werts bedeutet das, dass der erste Impedanz-Messwert und der zweite Impedanz-Messwert auf der Basis von Signalen bestimmt werden, die zeitlich versetzt voneinander abgegriffen werden.

Fig. 7 zeigt einen Sensor 2 gemäß einer gegenüber Fig. 6 modifizierten, weiteren beispielhaften Ausführungsform der Erfindung, wiederum teilweise in einem Blockdiagramm und teilweise in einem Schaltungsdiagramm dargestellt. Insbesondere bezieht sich die Modifikation auf die Signalverarbeitung in der Signal-Erfassungs- und Aufbereitungs-Schaltung 25. Im Allgemeinen sind entsprechende Komponenten mit den gleichen Bezugzeichen wie in Fig. 6 versehen. Für deren Beschreibung wird auf die obigen Ausführungen Bezug genommen.

Die Signal-Erfassungs- und Aufbereitungs-Schaltung 25 der Ausführungsform der Fig. 7 hat keine dritte Verstärkerschaltung 31 und keinen dritten Digital-AnalogWandler 34. Stattdessen ist die zweite Verstärkerschaltung 30 fest mit der ersten Messelektrode 11 verbunden und kann wahlweise mit der zweiten Messelektrode 12 oder der dritten Messelektrode 13 verbunden werden. Dazu ist ein Auswahlschalter 29d vorgesehen. Somit kann über die zweite Verstärkerschaltung 30 entweder die erste Spannung, d.h. die Spannung zwischen der ersten und zweiten Messelektrode 11, 12, oder die zweite Spannung, d.h. die Spannung zwischen der ersten und dritten Messelektrode 11, 13, an den zweiten Analog-Digital-Wandler 33 weitergeleitet werden. Für das Verfahren zum Bestimmen des die Impedanz der Suspension anzeigenden Werts bedeutet das, dass der erste Impedanz-Messwert und der zweite Impedanz-Messwert auf der Basis von Signalen bestimmt werden, die zeitlich versetzt voneinander abgegriffen werden.

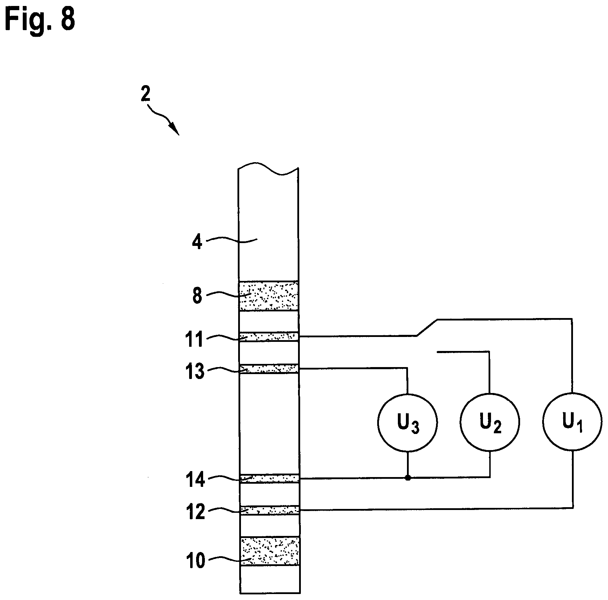

Fig. 8 zeigt einen Sensor 2 gemäß einer weiteren beispielhaften Ausführungsform der Erfindung in einer Seitenansicht. Der Sensor 2 der Fig. 8 hat, wie der Sensor 2 der Fig. 1, eine erste Erregerelektrode 8, eine zweite Erregerelektrode 10, eine erste Messelektrode 11, eine zweite Messelektrode 12, eine dritte Messelektrode 13 und eine vierte Messelektrode 14. Die sechs Elektroden sind in dem Sensor 2 der Fig. 8 genauso angeordnet wie in dem Sensor 2 der Fig. 1. Allerdings bilden die vier Messelektroden 11, 12, 13, 14 des Sensors 2 der Fig. 8 drei Paare von Messelektroden. Insbesondere besteht ein erstes Paar von Messelektroden aus **der ersten** Messelektrode 11 und der zweiten Messelektrode 12. Ein zweites Paar von Messelektroden besteht aus der ersten Messelektrode 11 und der vierten Messelektrode 14. Ein drittes Paar von Messelektroden besteht aus der dritten Messelektrode 13 und der vierten Messelektrode 14.

Im Betrieb werden eine erste Spannung U, an dem ersten Paar von Messelektroden 11 und 12, eine zweite Spannung $U_2$ an dem zweiten Paar von Messelektroden 11 und 14 und eine dritte Spannung $U_3$ an dem dritten Paar von Messelektroden 13 und 14 gemessen. Auf Basis des Erregerstroms, der ersten Spannung $U_1$, der zweiten Spannung $U_2$ und der dritten Spannung $U_3$ werden ein erster Impedanz-Messwert, ein zweiter Impedanz-Messwert und ein dritter Impedanz-Messwert bestimmt. Diese drei Impedanz-Messwerte werden zueinander in Beziehung gesetzt, um einen Impedanz-Wert für die Suspension zu bestimmen. Durch das Verwenden von drei Paaren von Messelektroden, welche aus einer Gesamtmenge von vier Messelektroden erstellt sind, können Störeinflüsse besonders gut eliminiert werden und ein besonders genauer Impedanz-Wert für die Suspension bestimmt werden.

Das Bestimmen des Impedanz-Werts Z kann dabei gemäß der folgenden Formel erfolgen:

$$Z^2 = k_2 \frac{\lambda_3 \left( G_{el}^{-1} \left( Z_{sig} \right)\big|_2 - G_{el}^{-1} \left( Z_{sig} \right)\big|_1 \right)}{\left( \lambda_1 - \lambda_2 \right)\left( \lambda_1 - \lambda_3 \right)\left( \lambda_2 - \lambda_3 \right)} +$$

$$+ k_2 \frac{\lambda_2 \left( G_{el}^{-1} \left( Z_{sig} \right)\big|_1 - G_{el}^{-1} \left( Z_{sig} \right)\big|_3 \right)}{\left( \lambda_1 - \lambda_2 \right)\left( \lambda_1 - \lambda_3 \right)\left( \lambda_2 - \lambda_3 \right)} +$$

$$+ k_2 \frac{\lambda_1 \left( G_{el}^{-1} \left( Z_{sig} \right)\big|_3 - G_{el}^{-1} \left( Z_{sig} \right)\big|_2 \right)}{\left( \lambda_1 - \lambda_2 \right)\left( \lambda_1 - \lambda_3 \right)\left( \lambda_2 - \lambda_3 \right)} .$$

Dabei bezeichnet $Z_{sig}|_1$ den ersten Impedanz-Messwert, $Z_{sig}|_2$ den zweiten Impedanz-Messwert und $Z_{sig}|_3$ den dritten Impedanz-Messwert. $G_{el}^{-1}$ bezeichnet eine Korrekturfunktion, die das Übertragungsverhalten der Messanordnung abbildet, wie oben beschrieben. Weiterhin bezeichnet $\lambda_1$ einen ersten Geometriefaktor, der die Messgeometrie des ersten Paars von Messelektroden 11, 12 abbildet, $\lambda_2$ einen zweiten Geometriefaktor, der die Messgeometrie des zweiten Paars von Messelektroden 11, 14 abbildet, und $\lambda_3$ einen dritten Geometriefaktor, der die Messgeometrie des dritten Paars von Messelektroden 13, 14 abbildet. Die Variable $k_2$ bezeichnet eine Proportionalitätskonstante.

Die oben bezüglich Fig. 2, 3, 6 und 7 gemachten Ausführungen zur gleichzeitigen bzw. zeitlich versetzten Signalverarbeitung gelten für den Sensor 2 der Fig. 8 analog. Beispielsweise können die vier Messelektroden mit zwei Verstärkerschaltungen und zwei Analog-Digital-Wandlern gekoppelt sein, so dass eine teilweise zeitgleiche und teilweise zeitversetzte Signalverarbeitung für die drei Messspannungen stattfindet. Die erste Spannung $U_1$ und die dritte Spannung $U_3$ können beispielsweise im Wesentlichen gleichzeitig gemessen werden, während die zweite Spannung $U_2$ danach gemessen wird. Es ist beispielsweise auch möglich, dass die vier Messelektroden mittels geeigneter Auswahlschalter mit einer einzigen Verstärkerschaltung und einem einzigen Analog-Digital-Wandler gekoppelt sind und die drei Spannungen nacheinander gemessen werden.

Es wird weiterhin betont, dass auch mehr als vier Messelektroden vorhanden sein können und dass mehr als drei Paare von Messelektroden gebildet werden können. Es können auch mehr als drei Impedanz-Messwerte für das Bestimmen des die Impedanz der Suspension anzeigenden Werts in Beziehung gesetzt werden.

Oben sind zwei Formeln angegeben, an Hand derer ein die Impedanz der Suspension anzeigender Wert für den Fall von zwei Impedanz-Messwerten und für den Fall von drei Impedanz-Messwerten bestimmt werden kann. Zu den Formeln werden im Folgenden einige Verständnishilfen bereitgestellt.

Wie oben beschrieben, ist das Ziel des Verfahrens das Bestimmen eines die Impedanz einer Suspension anzeigenden Werts. Dieser Wert kann beispielsweise direkt der Impedanz-Wert $Z$ sein.

In einer realen Messanordnung kann ein Impedanz-Messwert $Z_{sig}$ aus einer gemessenen Spannung $U_{sig}$ und einem gemessenen Strom $I_{sig}$ bestimmt werden. Allerdings kann dieser Impedanz-Messwert auf Grund der Signalverarbeitung in der Messanordnung von dem an den Messelektroden anliegenden Impedanz-Wert $Z_{mess}$ abweichen. Die Umrechnung zwischen dem Impedanz-Messwert $Z_{sig}$ und dem an den Messelektroden anliegenden Impedanz-Wert $Z_{mess}$ kann durch eine Übertragungsfunktion $G_{el}$ der Messanordnung bzw. eine dazu inverse Korrekturfunktion $G_{el}^{-1}$ ausgedrückt werden:

$$Z_{sig} \underset{G_{el}^{-1}}{\overset{G_{el}}{\rightleftarrows}} Z_{mess} \, .$$

In der Realität ist der an den Messelektroden anliegende Impedanz-Wert $Z_{mess}$ nicht die gesuchte Impedanz der Suspension, sondern setzt sich zusammen aus einem Impedanz-Wert $Z_{c.c.}$, der von der Messgeometrie abhängig ist, und einer Vielzahl von Störeinflüssen, wie z.B. parasitäre Kapazitäten, Doppelschichtbildung an den Elektrodenoberflächen, Übergangswiderstände, etc.. Die Störeinflüsse können insgesamt als parasitäre Einflüsse $Z_{par}$ bezeichnet werden können, die die Messung verfälschen. Die Gesamtheit der Störeinflüsse kann von einer Vielzahl von Parametern, wie z.B. Temperatur, Leitfähigkeit der Lösung, Frequenz des Erregerstroms, Ionenkonzentration in der Lösung, Material und Beschaffenheit der Elektroden, etc., abhängen. Man kann die Notation für diese Parameter zu einem Parameterset $\{\psi_i\}$ abkürzen. Die Parameter können auf verschiedene Art und Weise zu $Z_{par}$ beitragen und damit als verschiedene $Z_{par}^i\left(\{\psi_i\}\right)$ bezeichnet werden. Somit kann der an den Messelektroden anliegende Impedanz-Wert $Z_{mess}$ als Funktion $F$ gemäß der folgenden Beziehung ausgedrückt werden:

$$Z_{sig} \underset{G_{el}^{-1}}{\overset{G_{el}}{\rightleftarrows}} F\left(Z_{par}^i\left(\{\psi_i\}\right), Z_{c.c.}\right) \, .$$

Die Bezeichnung $Z_{c.c.}$ wird benutzt, weil die Impedanz einer Zellsuspension in guter Näherung durch die sogenannte Cole-Cole-Impedanz beschrieben werden kann.

Wie oben beschrieben, ist der Impedanz-Wert $Z_{c.c.}$ von der Messgeometrie abhängig. Die Messgeometrie wird hierin auch als Geometrie der Messzelle eines Paars von Messelektroden bezeichnet. Der gesuchte Impedanz-Wert $Z$ hängt mit dem Messgeometrie-abhängigen Impedanz-Wert $Z_{c.c.}$ über die Zellkonstante $\lambda$ zusammen. Es gilt der folgende Zusammenhang:

$$Z_{c.c.} = \frac{\lambda_j}{i\omega\epsilon_{cole-cole}} = \lambda_j Z \, .$$

Für die Zellkonstante $\lambda_j$ eines j-ten Paars von Messelektroden gilt:

$$\lambda_j = \frac{1}{\sigma_{L\ddot{o}sung}\,\Delta\phi^{(j)}} \int \vec{J}\,d\vec{A}\;,$$

wobei $\int \vec{J}\,d\vec{A}$ das Flächenintegral der Stromdichten und $\Delta\phi^{(j)}$ die Potentialdifferenz des j-ten Paars von Messelektroden sind.

Somit kann der an den Messelektroden anliegende Impedanz-Wert $Z_{mess}$ gemäß der folgenden Beziehung ausgedrückt werden:

$$Z_{sig} \overset{G_{el}}{\underset{G_{el}^{-1}}{\rightleftarrows}} F\left(Z_{par}^i(\{\psi_i\}), \lambda_j Z\right)\;.$$

Die Funktion $F$ kann als Polynom von $\lambda_j Z$ entwickelt werden. Somit ergibt sich:

$$Z_{sig} \overset{G_{el}}{\underset{G_{el}^{-1}}{\rightleftarrows}} \sum_n \frac{1}{n!} a_n\left(Z_{par}^i(\{\psi_i\})\right)\lambda_j^n Z^n\;.$$

Für das j-te Paar von Messelektroden ergibt sich die Entwicklung zweiter Ordnung zu:

$$G_{el}^{-1}\left(Z_{sig}\right)\big|_j = a_0\left(\left|Z_{par}^i\right|\right) + \lambda_j a_1\left(\left|Z_{par}^i\right|\right)Z + O(2)\;,$$

wobei $O(2)$ eine Abkürzung für den quadratischen Anteil der Polynomentwicklung ist.

Nimmt man an, dass die Vorfaktoren $a_n$ und die parasitären Einflüsse $Z_{par}^i$ für verschiedene Zellgeometrien identisch sind, und nimmt man weiter eine Reihenschaltung von parasitären Einflüssen und Zellenimpedanz an, so ergibt sich als Differenz für zwei Impedanz-Messwerte:

$$G_{el}^{-1}\left(Z_{sig}\right)\big|_1 - G_{el}^{-1}\left(Z_{sig}\right)\big|_2 = \left(\lambda_1 - \lambda_2\right)a_1\left(\left|Z_{par}^i\right|\right)Z + O(2)\;,$$

wobei $O(2)$ eine Abkürzung für alle quadratischen Anteile der Polynomentwicklungen ist.

Nimmt man weiterhin an, dass $O(2)$ sowie die Anteile noch höherer Ordnung vernachlässigbar sind und dass $a_1\left(\left|Z_{par}^i\right|\right)$ in guter Näherung eine Konstante ist, so gelangt man zu der oben genannten Formel für die Berechnung des die Impedanz der Suspension anzeigenden Werts $Z$ aus zwei Impedanz-Messwerten.

Es ist herausgefunden worden, dass unter den vorstehend beschriebenen Annahmen Messergebnisse sehr hoher Genauigkeit möglich sind.

Eine Weiterentwicklung der vorstehenden Überlegungen führt zu der weiteren oben genannten Formel für die Berechnung des die Impedanz der Suspension anzeigenden Werts $Z$ aus drei Impedanz-Messwerten.

Fig. 9 zeigt rein qualitativ den Verlauf 200 der Permittivität $\varepsilon$ einer Zellpopulation, aufgetragen gegenüber der Erregerfrequenz f. Der Verlauf 200 ist eine rein beispielhafte Kurve, die aus einer Mehrzahl von Werten für die Permittivität, welche durch das oben beschriebene Verfahren bestimmt worden sind, hergeleitet worden ist. Zum Beispiel kann der Verlauf 200 mittels eines Cole-Cole-Fittings aus der Mehrzahl von Permittivitätswerten hergeleitet worden sein.

Aus dem Verlauf 200 können folgendermaßen Charakteristika der Zellpopulation hergeleitet werden. In Fig. 9 ist qualitativ dargestellt, dass vor einer für das $\beta$-Dispersionsgebiet 202 charakteristischen Frequenz $f_{ch}$ ein Plateaugebiet 204 gelegen ist, in dem sich die Permittivität $\varepsilon$, verglichen mit dem Bereich um die charakteristische Frequenz $f_{ch}$, mit der Frequenz nur wenig ändert, und dass nach der charakteristischen Frequenz $f_{ch}$ ein weiteres Plateaugebiet 206 gelegen ist, welches von dem Plateaugebiet 204 vor der charakteristischen Frequenz $f_{ch}$ verschieden ist und in welchem sich die Permittivität $\varepsilon$, wiederum verglichen mit dem Bereich um die charakteristische Frequenz $f_{ch}$, ebenfalls nicht stark mit der Frequenz ändert.

Vergleicht man einen die Permittivität $\varepsilon$ repräsentierenden Permittivitätswert $\varepsilon_1$ bei einer Erregerfrequenz $f_1$ im Plateaugebiet 204 mit einem Permittivitätswert $\varepsilon_2$ bei einer Erregerfrequenz $f_2$ im Plateaugebiet 206, so lässt sich aus den

bei den Erregerfrequenzen $f_1$ und $f_2$ bestimmten Permittivitätswerten $\varepsilon_1$ bzw. $\varepsilon_2$ ein Unterschiedswert $\Delta\varepsilon$ der beiden Permittivitätswerte ermitteln. Der Unterschiedswert $\Delta\varepsilon$ ist dabei ein Maß für die Anzahl an in der Zellpopulation enthaltenen lebenden Zellen. Die mit zwei Punkten und drei Strichen angedeutete alternative Permittivitätskurve 210 würde zu einem betragsmäßig größeren $\Delta\varepsilon$ bei den jeweiligen Erregerfrequenzen $f_1$ und $f_2$ führen, was den Rückschluss zulässt, dass die Zellpopulation, für welche die Permittivitätskurve 210 erhalten wurde, mehr lebende Zellen in gleichem Volumen aufweist als die der Permittivitätskurve 200 zu Grunde liegende Zellpopulation.

Eine Veränderung der charakteristischen Frequenz $f_{ch}$ zeigt eine Änderung der Größe der Zellen oder ihrer Physiologie an. Eine Permittivitätskurve 220 mit zwei Punkten und einem Strich zeigt eine höhere charakteristische Frequenz $f_{ch}$ in Fig. 9. Die charakteristische Frequenz $f_{ch}$ kann aus dem Wendepunkt des Verlaufs 200 zwischen dem Plateaugebiet 204 und dem Plateaugebiet 206 bestimmt werden.

Die Steigung der Permittivitätskurve im Punkt ihrer charakteristischen Frequenz $f_{ch}$ ist ein Maß für die Zellgrößenverteilung, wobei zunehmende Steigung eine stärker heterogene Zellgrößenverteilung anzeigt und wobei flacher werdende Verläufe der Permittivitätskurve 200 am Ort der charakteristischen Frequenz $f_{ch}$ stärker homogene Zellgrößenverteilungen bedeuten.

Die in Fig. 9 gezeigten Permittivitätsverläufe können insbesondere die Verläufe der Realteile der bestimmten Permittivitätswerte sein.

In der beispielhaften Ausführungsform der Fig. 9 ist $f_1$ = 50 kHz und $f_2$ = 20 MHz.

Sensoren gemäß beispielhaften Ausführungsformen der Erfindung ermöglichen ein hochgenaues Bestimmen von Permittivitätswerten über einen solchen breiten Frequenzbereich, wodurch für viele Zellpopulationen das β-Dispersionsgebiet sehr weitreichend beschrieben werden kann. Insbesondere eignen sich der Sensor und das Verfahren zum Bestimmen eines die Impedanz einer Suspension anzeigenden Werts gemäß beispielhaften Ausführungsformen der Erfindung für Zellpopulationen mit einer Leitfähigkeit von wenigen 0.1mS/cm bis 100mS/cm und mit einer Permittivität von wenigen pF/cm bis einigen Hundert pF/cm.

Ein beispielhafter Anwendungsfall für den Sensor und das Verfahren zum Bestimmen eines die Impedanz einer Suspension anzeigenden Werts gemäß beispielhaften Ausführungsformen der Erfindung sind Gärprozesse, zum Beispiel beim Brauen von Getränken. Die Erfindung ist aber generell breit zum Bestimmen von die Impedanz einer Suspension anzeigenden Werten und für das nachgelagerte Bestimmen von Permittivitätswerten anwendbar.

Obwohl die Erfindung mit Bezug auf beispielhafte Ausführungsformen beschrieben worden ist, ist es für einen Fachmann ersichtlich, dass verschiedene Änderungen vorgenommen werden können, ohne den Bereich der Erfindung zu verlassen. Die Erfindung soll nicht durch die beschriebenen spezifischen Ausführungsformen beschränkt sein. Vielmehr enthält sie alle Ausführungsformen, die unter die angehängten Patentansprüche fallen.

**Patentansprüche**

1. Verfahren zum Bestimmen eines die Impedanz einer Suspension anzeigenden Werts im Rahmen einer Impedanzspektroskopie, aufweisend die folgenden Schritte:

Erzeugen eines mit einer Erregerfrequenz oszillierenden Erregerstroms durch die Suspension, wobei der Erregerstrom durch die Suspension mittels eines Paars von Erregerelektroden (8, 10) erzeugt wird,
Bestimmen eines ersten Impedanz-Messwerts auf Basis des Erregerstroms und einer ersten Spannung an einem ersten Paar von Messelektroden (11, 12),
Bestimmen eines zweiten Impedanz-Messwerts auf Basis des Erregerstroms und einer zweiten Spannung an einem zweiten Paar von Messelektroden (11, 13; 13, 14), wobei das erste Paar von Messelektroden und das zweite Paar von Messelektroden unterschiedliche Paare von Messelektroden sind,

Bestimmen des die Impedanz der Suspension anzeigenden Werts durch In-Beziehung-Setzen des ersten Impedanz-Messwerts und des zweiten Impedanz-Messwerts,
wobei das Bestimmen des die Impedanz der Suspension anzeigenden Werts eine Differenzbildung eines ersten angepassten Impedanz-Werts und eines zweiten angepassten Impedanz-Werts aufweist, wobei der erste angepasste Impedanz-Wert und der zweite angepasste Impedanz-Wert durch Anwenden einer Korrekturfunktion auf den ersten Impedanz-Messwert und den zweiten Impedanz-Messwert erhalten werden, wobei die Korrekturfunktion das Übertragungsverhalten der Messanordnung abbildet,
wobei das In-Beziehung-Setzen des ersten Impedanz-Messwerts und des zweiten Impedanz-Messwerts gemäß folgender Formel erfolgt:

$$Z = k \frac{1}{(\lambda_1 - \lambda_2)} \left( G_{el}^{-1} \left( Z_{sig} \right) \Big|_1 - G_{el}^{-1} \left( Z_{sig} \right) \Big|_2 \right),$$

wobei $Z_{sig}|_1$ den ersten Impedanz-Messwert bezeichnet, $Z_{sig}|_2$ den zweiten Impedanz-Messwert bezeichnet, $G_{el}^{-1}$ die Korrekturfunktion bezeichnet, die das Übertragungsverhalten der Messanordnung abbildet, $\lambda_1$ einen ersten Geometriefaktor bezeichnet, der die Messgeometrie des ersten Paars von Messelektroden abbildet, $\lambda_2$ einen zweiten Geometriefaktor bezeichnet, der die Messgeometrie des zweiten Paars von Messelektroden abbildet, und k eine Proportionalitätskonstante bezeichnet.

2. Verfahren nach Anspruch 1,

wobei das erste Paar von Messelektroden aus einer ersten Messelektrode (11) und einer zweiten Messelektrode (12) besteht und wobei das zweite Paar von Messelektroden aus der ersten Messelektrode (11) und einer dritten Messelektrode (13) besteht,
oder
wobei das erste Paar von Messelektroden aus einer ersten Messelektrode (11) und einer zweiten Messelektrode (12) besteht und wobei das zweite Paar von Messelektroden aus einer dritten Messelektrode (13) und einer vierten Messelektrode (14) besteht.

3. Verfahren nach einem der vorhergehenden Ansprüche,

weiterhin aufweisend:

Messen der ersten Spannung an dem ersten Paar von Messelektroden, und
Messen der zweiten Spannung an dem zweiten Paar von Messelektroden,
wobei das Messen der ersten Spannung und das Messen der zweiten Spannung im Wesentlichen zeitgleich erfolgen;
oder

weiterhin aufweisend:

Messen der ersten Spannung an dem ersten Paar von Messelektroden, und
Messen der zweiten Spannung an dem zweiten Paar von Messelektroden,
wobei das Messen der ersten Spannung und das Messen der zweiten Spannung zeitlich versetzt erfolgen.

4. Verfahren nach einem der vorhergehenden Ansprüche, wobei das Bestimmen des ersten Impedanz-Messwerts und das Bestimmen des zweiten Impedanz-Messwerts umfasst:

Abtasten des Erregerstroms,
Abtasten der ersten Spannung, und
Abtasten der zweiten Spannung;
wobei das Verfahren insbesondere weiterhin die folgenden Schritte aufweist:

Einstellen einer ersten Abtastrate für das Abtasten des Erregerstroms,
Einstellen einer zweiten Abtastrate für das Abtasten der ersten Spannung, und
Einstellen einer dritten Abtastrate für das Abtasten der zweiten Spannung,
wobei die erste Abtastrate, die zweite Abtastrate und die dritte Abtastrate mindestens auf das 4-fache der Erregerfrequenz des Erregerstroms, insbesondere im Wesentlichen auf das 4-fache der Erregerfrequenz des Erregerstroms, eingestellt werden.

5. Verfahren nach Anspruch 4, wobei der Schritt des Bestimmens des ersten Impedanz-Messwerts ein Durchführen einer ersten komplexen Fourier-Transformation auf Basis der Abtastwerte des Erregerstroms und der Abtastwerte der ersten Spannung aufweist und wobei der Schritt des Bestimmens des zweiten Impedanz-Messwerts ein Durchführen einer zweiten komplexen Fourier-Transformation auf Basis der Abtastwerte des Erregerstroms und der Abtastwerte der zweiten Spannung aufweist.

6. Verfahren nach einem der vorhergehenden Ansprüche, weiterhin aufweisend:

Bestimmen eines dritten Impedanz-Messwerts auf Basis des Erregerstroms und einer dritten Spannung an einem dritten Paar von Messelektroden, und

Bestimmen des die Impedanz der Suspension anzeigenden Werts durch In-Beziehung-Setzen des ersten Impedanz-Messwerts, des zweiten Impedanz-Messwerts und des dritten Impedanz-Messwerts; wobei das Bestimmen des die Impedanz der Suspension anzeigenden Werts eine erste Differenzbildung des ersten angepassten Impedanz-Werts und des zweiten angepassten Impedanz-Werts und eine zweite Differenzbildung des ersten angepassten Impedanz-Werts und eines dritten angepassten Impedanz-Werts und eine dritte Differenzbildung des zweiten angepassten Impedanz-Werts und des dritten angepassten Impedanz-Werts aufweist, wobei der erste angepasste Impedanz-Wert, der zweite angepasste Impedanz-Wert und der dritte angepasste Impedanz-Wert durch Anwenden der Korrekturfunktion auf den ersten Impedanz-Messwert, den zweiten Impedanz-Messwert und den dritten Impedanz-Messwert erhalten werden.

7.  Verfahren nach Anspruch 6, wobei das Bestimmen des die Impedanz der Suspension anzeigenden Werts eine erste Differenzbildung des ersten Geometriefaktors und des zweiten Geometriefaktors und eine zweite Differenzbildung des ersten Geometriefaktors und eines dritten Geometriefaktors und eine dritte Differenzbildung des zweiten Geometriefaktors und des dritten Geometriefaktors aufweist, wobei der dritte Geometriefaktor die Messgeometrie des dritten Paars von Messelektroden abbildet;

wobei das Bestimmen des die Impedanz der Suspension anzeigenden Werts insbesondere gemäß folgender Formel erfolgt:

$$Z^2 = k_2 \frac{\lambda_3 \left( G_{el}^{-1}\left(Z_{sig}\right)\big|_2 - G_{el}^{-1}\left(Z_{sig}\right)\big|_1 \right)}{\left(\lambda_1 - \lambda_2\right)\left(\lambda_1 - \lambda_3\right)\left(\lambda_2 - \lambda_3\right)} +$$

$$+ k_2 \frac{\lambda_2 \left( G_{el}^{-1}\left(Z_{sig}\right)\big|_1 - G_{el}^{-1}\left(Z_{sig}\right)\big|_3 \right)}{\left(\lambda_1 - \lambda_2\right)\left(\lambda_1 - \lambda_3\right)\left(\lambda_2 - \lambda_3\right)} +$$

$$+ k_2 \frac{\lambda_1 \left( G_{el}^{-1}\left(Z_{sig}\right)\big|_3 - G_{el}^{-1}\left(Z_{sig}\right)\big|_2 \right)}{\left(\lambda_1 - \lambda_2\right)\left(\lambda_1 - \lambda_3\right)\left(\lambda_2 - \lambda_3\right)} \quad,$$

wobei $Z_{sig}|_3$ den dritten Impedanz-Messwert bezeichnet, $\lambda_3$ einen dritten Geometriefaktor bezeichnet, der die Messgeometrie des dritten Paars von Messelektroden abbildet, und $k_2$ eine Proportionalitätskonstante bezeichnet.

8.  Verfahren zum Bestimmen eines die Impedanz einer Suspension anzeigenden Werts im Rahmen einer Impedanzspektroskopie, aufweisend die folgenden Schritte:

Erzeugen einer mit einer Erregerfrequenz oszillierenden, an der Suspension anliegenden Erregerspannung, wobei die Erregerspannung durch die Suspension mittels eines Paars von Erregerelektroden (8, 10) erzeugt wird,

Bestimmen eines ersten Impedanz-Messwerts auf Basis der Erregerspannung und eines ersten Stroms durch ein erstes Paar von Messelektroden,

Bestimmen eines zweiten Impedanz-Messwerts auf Basis der Erregerspannung und eines zweiten Stroms durch ein zweites Paar von Messelektroden, wobei das erste Paar von Messelektroden und das zweite Paar von Messelektroden unterschiedliche Paare von Messelektroden sind,

Bestimmen des die Impedanz der Suspension anzeigenden Werts durch In-Beziehung-Setzen des ersten Impedanz-Messwerts und des zweiten Impedanz-Messwerts,

wobei das Bestimmen des die Impedanz der Suspension anzeigenden Werts eine Differenzbildung eines ersten angepassten Impedanz-Werts und eines zweiten angepassten Impedanz-Werts aufweist, wobei der erste angepasste Impedanz-Wert und der zweite angepasste Impedanz-Wert durch Anwenden einer Korrekturfunktion auf den ersten Impedanz-Messwert und den zweiten Impedanz-Messwert erhalten werden, wobei die Korrekturfunktion vorzugsweise das Übertragungsverhalten der Messanordnung abbildet,
wobei das In-Beziehung-Setzen des ersten Impedanz-Messwerts und des zweiten Impedanz-Messwerts gemäß folgender Formel erfolgt:

$$Z = k \, \frac{1}{(\lambda_1 - \lambda_2)} \left( G_{el}^{-1}\left(Z_{sig}\right)\big|_1 - G_{el}^{-1}\left(Z_{sig}\right)\big|_2 \right)$$

,

wobei $Z_{sig}|_1$ den ersten Impedanz-Messwert bezeichnet, $Z_{sig}|_2$ den zweiten Impedanz-Messwert bezeichnet, $G_{el}^{-1}$ die Korrekturfunktion bezeichnet, die das Übertragungsverhalten der Messanordnung abbildet, $\lambda_1$ einen ersten Geometriefaktor bezeichnet, der die Messgeometrie des ersten Paars von Messelektroden abbildet, $\lambda_2$ einen zweiten Geometriefaktor bezeichnet, der die Messgeometrie des zweiten Paars von Messelektroden abbildet, und k eine Proportionalitätskonstante bezeichnet.

9. Verfahren zum Herleiten von mindestens einer charakteristischen Eigenschaft einer Suspension, aufweisend die folgenden Schritte:

mehrmaliges Durchführen des Verfahrens zum Bestimmen eines die Impedanz einer Suspension anzeigenden Werts gemäß einem der vorhergehenden Ansprüche unter Verwendung von einer Mehrzahl von unterschiedlichen Erregerfrequenzen und Bestimmen einer Mehrzahl von die Impedanz der Suspension anzeigenden Werten für die Mehrzahl von unterschiedlichen Erregerfrequenzen,
Herleiten einer Mehrzahl von die Permittivität der Suspension anzeigenden Werten auf Basis der Mehrzahl von die Impedanz der Suspension anzeigenden Werten, und
Herleiten der mindestens einen charakteristischen Eigenschaft der Suspension durch In-Beziehung-Setzen der Mehrzahl von die Permittivität der Suspension anzeigenden Werten.

10. Verfahren nach Anspruch 9,

wobei das genannte Verfahren zum Bestimmen eines die Impedanz einer Suspension anzeigenden Werts für zwischen 2 und 50 unterschiedliche Erreger-frequenzen, insbesondere für zwischen 10 und 40 unterschiedliche Erregerfrequenzen, weiter insbesondere für zwischen 20 und 30 unterschiedliche Erregerfrequenzen, durchgeführt wird;
und/oder
wobei die unterschiedlichen Erregerfrequenzen aus einem Frequenzbereich von 100 kHz bis 10 MHz, insbesondere aus einem Frequenzbereich von 50 kHz bis 20 MHz, stammen.

11. Verfahren nach Anspruch 9 oder 10,

wobei das Herleiten der mindestens einen charakteristischen Eigenschaft der Suspension ein Erstellen eines Verlaufs der die Permittivität der Suspension anzeigenden Werte über die unterschiedlichen Erregerfrequenzen beinhaltet;
und/oder
wobei die Suspension eine Zellpopulation ist und wobei die mindestens eine charakteristische Eigenschaft der Suspension mindestens eine Eigenschaft von Anzahl der lebenden Zellen, Größe der Zellen und Homogenität der Zellen umfasst.

12. Sensor (2) zum Bestimmen eines die Impedanz einer Suspension anzeigenden Werts, aufweisend:

eine Oszillatorschaltung (16),
ein Paar von Erregerelektroden (8, 10), die mit der Oszillatorschaltung gekoppelt sind, wobei mittels der Oszillatorschaltung über das Paar von Erregerelektroden ein mit einer Erregerfrequenz oszillierender Erregerstrom durch die Suspension erzeugbar ist,
mindestens drei Messelektroden zum Messen einer ersten Spannung in der Suspension zwischen einem ersten Paar der mindestens drei Messelektroden und einer zweiten Spannung in der Suspension zwischen einem zweiten Paar der mindestens drei Messelektroden, wobei das erste Paar von Messelektroden und das zweite Paar von Messelektroden unterschiedliche Paare von Messelektroden sind, und
eine Datenverarbeitungseinrichtung (40), die konfiguriert ist, einen ersten Impedanz-Messwert auf Basis des Erregerstroms und der ersten Spannung zu bestimmen, einen zweiten Impedanz-Messwert auf Basis des Erregerstroms und der zweiten Spannung zu bestimmen, und den die Impedanz der Suspension anzeigenden Wert durch In-Beziehung-Setzen des ersten Impedanz-Messwerts und des zweiten Impedanz-Messwerts zu bestimmen;

wobei die Datenverarbeitungseinrichtung (40) konfiguriert ist, den die Impedanz der Suspension anzeigenden Wert mittels einer Differenzbildung eines ersten angepassten Impedanz-Werts und eines zweiten angepassten Impedanz-Werts zu bestimmen, wobei die Datenverarbeitungseinrichtung konfiguriert ist, den ersten angepassten Impedanz-Wert und den zweiten angepassten Impedanz-Wert durch Anwenden einer Korrekturfunktion auf den ersten Impedanz-Messwert und den zweiten Impedanz-Messwert zu erhalten, wobei die Korrekturfunktion das Übertragungsverhalten der Messanordnung abbildet,

wobei die Datenverarbeitungseinrichtung (40) konfiguriert ist, den die Impedanz der Suspension anzeigenden Wert gemäß folgender Formel zu bestimmen:

$$Z = k \, \frac{1}{(\lambda_1 - \lambda_2)} \left( G_{el}^{-1}\left(Z_{sig}\right)\big|_1 - G_{el}^{-1}\left(Z_{sig}\right)\big|_2 \right)$$

,

wobei $Z_{sig}|_1$ den ersten Impedanz-Messwert bezeichnet, $Z_{sig}|_2$ den zweiten Impedanz-Messwert bezeichnet, $G_{el}^{-1}$ die Korrekturfunktion bezeichnet, die das Übertragungsverhalten der Messanordnung abbildet, $\lambda_1$ einen ersten Geometriefaktor bezeichnet, der die Messgeometrie des ersten Paars von Messelektroden abbildet, $\lambda_2$ einen zweiten Geometriefaktor bezeichnet, der die Messgeometrie des zweiten Paars von Messelektroden abbildet, und k eine Proportionalitätskonstante bezeichnet.

13. Sensor (2) nach Anspruch 12, wobei die mindestens drei Messelektroden zwischen dem Paar von Erregerelektroden (8, 10) angeordnet sind.

14. Sensor (2) nach Anspruch 12 oder 13,

wobei die mindestens drei Messelektroden mindestens vier Messelektroden sind, wobei das erste Paar der mindestens vier Messelektroden aus einer ersten Messelektrode (11) und einer zweiten Messelektrode (12) besteht und wobei das zweite Paar der mindestens vier Messelektroden aus einer dritten Messelektrode (13) und einer vierten Messelektrode (14) besteht;

wobei die dritte und vierte Messelektrode (13, 14) insbesondere zwischen der ersten und zweiten Messelektrode (11, 12) angeordnet sind und/oder wobei die dritte und vierte Messelektrode (13, 14) insbesondere an einer anderen Seite des Sensors als die erste und zweite Messelektrode (11, 12) angeordnet sind.

15. Sensor (2) nach einem der Ansprüche 12 bis 14, wobei die Oszillatorschaltung (16) über einen Transformator (22) mit dem Paar von Erregerelektroden (8, 10) gekoppelt ist, wobei der Transformator (22) insbesondere eine parallele Kapazität von 0,5 bis 10 pF, weiter insbesondere von 1 bis 5 pF, hat.

16. Sensor (2) zum Bestimmen eines die Impedanz einer Suspension anzeigenden Werts, aufweisend:

eine Oszillatorschaltung (16),

ein Paar von Erregerelektroden (8, 10), die mit der Oszillatorschaltung gekoppelt sind, wobei mittels der Oszillatorschaltung über das Paar von Erregerelektroden eine mit einer Erregerfrequenz oszillierende, an der Suspension anliegende Erregerspannung erzeugbar ist,

mindestens drei Messelektroden zum Messen eines ersten Stroms in der Suspension zwischen einem ersten Paar der mindestens drei Messelektroden und eines zweiten Stroms in der Suspension zwischen einem zweiten Paar der mindestens drei Messelektroden, wobei das erste Paar von Messelektroden und das zweite Paar von Messelektroden unterschiedliche Paare von Messelektroden sind, und

eine Datenverarbeitungseinrichtung (40), die konfiguriert ist, einen ersten Impedanz-Messwert auf Basis der Erregerspannung und des ersten Stroms zu bestimmen, einen zweiten Impedanz-Messwert auf Basis der Erregerspannung und des zweiten Stroms zu bestimmen, und den die Impedanz der Suspension anzeigenden Wert durch In-Beziehung-Setzen des ersten Impedanz-Messwerts und des zweiten Impedanz-Messwerts zu bestimmen;

wobei die Datenverarbeitungseinrichtung (40) konfiguriert ist, den die Impedanz der Suspension anzeigenden Wert mittels einer Differenzbildung eines ersten angepassten Impedanz-Werts und eines zweiten angepassten Impedanz-Werts zu bestimmen, wobei die Datenverarbeitungseinrichtung konfiguriert ist, den ersten angepassten Impedanz-Wert und den zweiten angepassten Impedanz-Wert durch Anwenden einer Korrekturfunktion auf den ersten Impedanz-Messwert und den zweiten Impedanz-Messwert zu erhalten, wobei die Korrekturfunktion das Übertragungsverhalten der Messanordnung abbildet,

wobei die Datenverarbeitungseinrichtung (40) konfiguriert ist, den die Impedanz der Suspension anzeigenden Wert gemäß folgender Formel zu bestimmen:

$$Z = k \frac{1}{(\lambda_1 - \lambda_2)} \left( G_{el}^{-1}(Z_{sig})\big|_1 - G_{el}^{-1}(Z_{sig})\big|_2 \right),$$

wobei $Z_{sig}\big|_1$ den ersten Impedanz-Messwert bezeichnet, $Z_{sig}\big|_2$ den zweiten Impedanz-Messwert bezeichnet, $G_{el}^{-1}$ die Korrekturfunktion bezeichnet, die das Übertragungsverhalten der Messanordnung abbildet, $\lambda_1$ einen ersten Geometriefaktor bezeichnet, der die Messgeometrie des ersten Paars von Messelektroden abbildet, $\lambda_2$ einen zweiten Geometriefaktor bezeichnet, der die Messgeometrie des zweiten Paars von Messelektroden abbildet, und k eine Proportionalitätskonstante bezeichnet.

17. Computerprogramm, welches Programmanweisungen enthält, die bei Ausführung auf einer Datenverarbeitungsanlage ein Verfahren nach einem der Ansprüche 1 bis 11 durchführen, wobei die einzelnen Schritte des Verfahrens durch die Programmanweisungen veranlasst werden und durch Komponenten eines Sensors ausgeführt werden oder in der Datenverarbeitungsanlage selbst ausgeführt werden.

## Claims

1. A method for determining a value indicative of the impedance of a suspension in the framework of an impedance spectroscopy, comprising the following steps:

generating an excitation current through the suspension, the excitation current oscillating at an excitation frequency, wherein the excitation current through the suspension is generated by means of a pair of excitation electrodes (8, 10),

determining a first impedance measurement value on the basis of the excitation current and a first voltage at a first pair of measurement electrodes (11, 12),

determining a second impedance measurement value on the basis of the excitation current and a second voltage at a second pair of measurement electrodes (11, 13; 13, 14), wherein the first pair of measurement electrodes and the second pair of measurement electrodes are different pairs of measurements electrodes,

determining the value indicative of the impedance of the suspension by relating the first impedance measurement value and the second impedance measurement value,

wherein determining the value indicative of the impedance of the suspension comprises determining the difference between a first adjusted impedance value and a second adjusted impedance value, wherein the first adjusted impedance value and the second adjusted impedance value are obtained by applying a correction function to the first impedance measurement value and the second impedance measurement value, the correction function representing the transmission behavior of the measurement arrangement,

wherein the relating of the first impedance measurement value and the second impedance measurement value is carried out according to the following formula:

$$Z = k \frac{1}{(\lambda_1 - \lambda_2)} \left( G_{el}^{-1}(Z_{sig})\big|_1 - G_{el}^{-1}(Z_{sig})\big|_2 \right),$$

wherein $Z_{sig}\big|_1$ denotes the first impedance measurement value, $Z_{sig}\big|_2$ denotes the second impedance measure-ment value, $G_{el}^{-1}$ denotes the correction function representing the transmission behavior of the measurement arrangement, $\lambda_1$ denotes a first geometry factor representing the measurement geometry of the first pair of measurement electrodes, $\lambda_2$ denotes a second geometry factor representing the measurement geometry of the second pair of measurement electrodes, and k denotes a proportionality constant.

2. The method according to claim 1,

wherein said first pair of measurement electrodes comprises a first measurement electrode (11) and a second measurement electrode (12), and wherein said second pair of measurement electrodes comprises said first measurement electrode (11) and a third measurement electrode (13),
or
wherein the first pair of measurement electrodes comprises a first measurement electrode (11) and a second measurement electrode (12), and wherein the second pair of measurement electrodes comprises a third measurement electrode (13) and a fourth measurement electrode (14).

3. The method according to any of the preceding claims,

   further comprising:

   measuring the first voltage at the first pair of measurement electrodes, and
   measuring the second voltage at the second pair of measurement electrodes,
   wherein measuring the first voltage and measuring the second voltage are performed substantially simultaneously,
   or

   further comprising:

   measuring the first voltage at the first pair of measurement electrodes, and
   measuring the second voltage at the second pair of measurement electrodes,
   wherein measuring the first voltage and measuring the second voltage are performed in a time-shifted manner.

4. The method according to any of the preceding claims,
   wherein determining the first impedance measurement value and determining the second impedance measurement value comprises:

   sampling the excitation current,
   sampling the first voltage, and
   sampling the second voltage;
   wherein the method in particular further comprises the steps of:

   setting a first sampling rate for sampling the excitation current,
   setting a second sampling rate for sampling the first voltage, and
   setting a third sampling rate for sampling the second voltage,
   wherein the first sampling rate, the second sampling rate and the third sampling rate are set to at least 4 times the excitation frequency of the excitation current, in particular to substantially 4 times the excitation frequency of the excitation current.

5. The method according to claim 4,
   wherein the step of determining the first impedance measurement value comprises performing a first complex Fourier transform on the basis of the sampling values of the excitation current and the sampling values of the first voltage, and wherein the step of determining the second impedance measurement value comprises performing a second complex Fourier transform on the basis of the sampling values of the excitation current and the sampling values of the second voltage.

6. The method according to any of the preceding claims, further comprising:

   determining a third impedance measurement value on the basis of the excitation current and a third voltage at a third pair of measurement electrodes, and determining the value indicative of the impedance of the suspension by relating the first impedance measurement value, the second impedance measurement value, and the third impedance measurement value;
   wherein determining the value indicative of the impedance of the suspension comprises determining a first difference between the first adjusted impedance value and the second adjusted impedance value and determining a second difference between the first adjusted impedance value and a third adjusted impedance value and determining a third difference between the second adjusted impedance value and the third adjusted impedance value, wherein the first adjusted impedance value, the second adjusted impedance value and
   the third adjusted impedance value are obtained by applying the correction function to the first impedance measurement value, the second impedance measurement value and the third impedance measurement value.

7. The method according to claim 6,

wherein determining the value indicative of the impedance of the suspension comprises determining a first difference between the first geometry factor and the second geometry factor and determining a second difference between the first geometry factor and a third geometry factor and determining a third difference between the second geometry factor and the third geometry factor, wherein the third geometry factor represents the measurement geometry of the third pair of measurement electrodes;

wherein determining the value indicative of the impedance of the suspension is in particular carried out according to the following formula:

$$Z^2 = k_2 \frac{\lambda_3\left(G_{el}^{-1}\left(Z_{sig}\right)\big|_2 - G_{el}^{-1}\left(Z_{sig}\right)\big|_1\right)}{\left(\lambda_1 - \lambda_2\right)\left(\lambda_1 - \lambda_3\right)\left(\lambda_2 - \lambda_3\right)} +$$

$$+ k_2 \frac{\lambda_2\left(G_{el}^{-1}\left(Z_{sig}\right)\big|_1 - G_{el}^{-1}\left(Z_{sig}\right)\big|_3\right)}{\left(\lambda_1 - \lambda_2\right)\left(\lambda_1 - \lambda_3\right)\left(\lambda_2 - \lambda_3\right)} +$$

$$+ k_2 \frac{\lambda_1\left(G_{el}^{-1}\left(Z_{sig}\right)\big|_3 - G_{el}^{-1}\left(Z_{sig}\right)\big|_2\right)}{\left(\lambda_1 - \lambda_2\right)\left(\lambda_1 - \lambda_3\right)\left(\lambda_2 - \lambda_3\right)} \quad ,$$

wherein $Z_{sig}|_3$ denotes the third impedance measurement value, $\lambda_3$ denotes a third geometry factor that represents the measurement geometry of the third pair of measurement electrodes, and $k_2$ denotes a proportionality constant.

8. A method for determining a value indicative of the impedance of a suspension in the framework of an impedance spectroscopy, comprising the following steps:

generating an excitation voltage, oscillating at an excitation frequency, applied to the suspension, wherein the excitation voltage through the suspension is generated by means of a pair of excitation electrodes (8, 10),

determining a first impedance measurement value on the basis of the excitation voltage and a first current through a first pair of measurement electrodes, determining a second impedance measurement value on the basis of the excitation voltage and a second current through a second pair of measurement electrodes, wherein the first pair of measurement electrodes and the second pair of measurement electrodes are different pairs of measurements electrodes,

determining the value indicative of the impedance of the suspension by relating the first impedance measurement value and the second impedance measurement value,

wherein determining the value indicative of the impedance of the suspension comprises determining the difference between a first adjusted impedance value and a second adjusted impedance value, wherein the first adjusted impedance value and the second adjusted impedance value are obtained by applying a correction function to the first impedance measurement value and the second impedance measurement value, the correction function preferably representing the transmission behavior of the measurement arrangement,

wherein the relating of the first impedance measurement value and the second impedance measurement value is carried out according to the following formula:

$$Z = k \frac{1}{\left(\lambda_1 - \lambda_2\right)}\left(G_{el}^{-1}\left(Z_{sig}\right)\big|_1 - G_{el}^{-1}\left(Z_{sig}\right)\big|_2\right) \quad ,$$

wherein $Z_{sig}|_1$ denotes the first impedance measurement value, $Z_{sig}|_2$ denotes the second impedance measurement value, $G_{el}^{-1}$ denotes the correction function representing the transmission behavior of the measurement

arrangement, $\lambda_1$ denotes a first geometry factor representing the measurement geometry of the first pair of measurement electrodes, $\lambda_2$ denotes a second geometry factor representing the measurement geometry of the second pair of measurement electrodes, and k denotes a proportionality constant.

9. A method for deriving at least one characteristic property of a suspension, comprising the steps of:

performing the method for determining a value indicative of the impedance of a suspension according to any of the preceding claims a plurality of times, using a plurality of different excitation frequencies and determining a plurality of values indicative of the impedance of the suspension for the plurality of different excitation frequencies,
deriving a plurality of values indicative of the permittivity of the suspension based on the plurality of values indicative of the impedance of the suspension, and
deriving the at least one characteristic property of the suspension by relating the plurality of values indicative of the permittivity of the suspension.

10. The method according to claim 9,

wherein said method for determining a value indicative of the impedance of a suspension is performed for between 2 and 50 different excitation frequencies, in particular for between 10 and 40 different excitation frequencies, further in particular for between 20 and 30 different excitation frequencies; and/or
wherein the different excitation frequencies are from a frequency range from 100 kHz to 10 MHz, in particular from a frequency range from 50 kHz to 20 MHz.

11. The method according to claim 9 or 10,

wherein deriving the at least one characteristic property of the suspension includes generating a curve of the values indicative of the permittivity of the suspension over the different excitation frequencies;
and/or
wherein the suspension is a cell population and wherein the at least one characteristic property of the suspension comprises at least one property of number of living cells, size of the cells and homogeneity of the cells.

12. A sensor (2) for determining a value indicative of the impedance of a suspension, comprising:

an oscillator circuit (16),
a pair of excitation electrodes (8, 10) coupled to the oscillator circuit, wherein
an excitation current through the suspension, oscillating at an excitation frequency, can be generated across the pair of excitation electrodes by means of the oscillator circuit,
at least three measurement electrodes for measuring a first voltage in the suspension between a first pair of the at least three measurement electrodes and a second voltage in the suspension between a second pair of the at least three measurement electrodes, wherein the first pair of measurement electrodes and the second pair of measurement electrodes are different pairs of measurements electrodes, and
a data processing device (40) configured to determine a first impedance measurement value on the basis of the excitation current and the first voltage, to determine a second impedance measurement value on the basis of the excitation current and the second voltage, and to determine the value indicative of the impedance of the suspension by relating the first impedance measurement value and the second impedance measurement value;
wherein the data processing device (40) is configured to determine the value indicative of the impedance of the suspension via determining the difference between a first adjusted impedance value and a second adjusted impedance value, wherein the data processing device is configured to determine the first adjusted impedance value and the second adjusted impedance value by applying a correction function to the first impedance measurement value and the second impedance measurement value, wherein the correction function represents the transmission behavior of the measurement arrangement,
wherein the data processing device (40) is configured to determine the value indicative of the impedance of the suspension according to the following formula:

$$Z = k \, \frac{1}{(\lambda_1 - \lambda_2)} \left( G_{el}^{-1}(Z_{sig})\big|_1 - G_{el}^{-1}(Z_{sig})\big|_2 \right)$$

,

wherein $Z_{sig}|_1$ denotes the first impedance measurement value, $Z_{sig}|_2$ denotes the second impedance measure-

ment value, $G_{el}^{-1}$ denotes the correction function representing the transmission behavior of the measurement arrangement, $\lambda_1$ denotes a first geometry factor representing the measurement geometry of the first pair of the at least three measurement electrodes, $\lambda_2$ denotes a second geometry factor representing the measurement geometry of the second pair of the at least three measurement electrodes, and k denotes a proportionality constant.

13. The sensor (2) according to claim 12,
    wherein the at least three measurement electrodes are arranged between the pair of excitation electrodes (8, 10).

14. The sensor (2) according to claim 12 or 13,

    wherein the at least three measurement electrodes are at least four measurement electrodes, wherein the first pair of the at least four measurement electrodes comprises a first measurement electrode (11) and a second measurement electrode (12) and wherein the second pair of the at least four measurement electrodes comprises a third measurement electrode (13) and a fourth measurement electrode (14);
    wherein the third and fourth measurement electrodes (13, 14) are in particular arranged between the first and second measurement electrodes (11, 12) and/or wherein the third and fourth measurement electrodes (13, 14) are in particular arranged on a different side of the sensor than the first and second measurement electrodes (11, 12).

15. The sensor (2) according to any of claims 12 to 14,
    wherein the oscillator circuit (16) is coupled to the pair of excitation electrodes (8, 10) via a transformer (22), wherein the transformer (22) in particular has a parallel capacitance of 0.5 to 10 pF, further in particular of 1 to 5 pF.

16. A sensor (2) for determining a value indicative of the impedance of a suspension, comprising:

    an oscillator circuit (16),
    a pair of excitation electrodes (8, 10) coupled to the oscillator circuit, wherein an excitation voltage, oscillating at an excitation frequency, applied to the suspension can be generated across the pair of excitation electrodes by means of the oscillator circuit,
    at least three measurement electrodes for measuring a first current in the suspension between a first pair of the at least three measurement electrodes and a second current in the suspension between a second pair of the at least three measurement electrodes, wherein the first pair of measurement electrodes and the second pair of measurement electrodes are different pairs of measurements electrodes, and
    a data processing device (40) configured to determine a first impedance measurement value on the basis of the excitation voltage and the first current, to determine a second impedance measurement value on the basis of the excitation voltage and the second current, and to determine the value indicative of the impedance of the suspension by relating the first impedance measurement value and the second impedance measurement value;
    wherein the data processing device (40) is configured to determine the value indicative of the impedance of the suspension via determining the difference between a first adjusted impedance value and a second adjusted impedance value, wherein the data processing device is configured to determine the first adjusted impedance value and the second adjusted impedance value by applying a correction function to the first impedance measurement value and the second impedance measurement value, wherein the correction function represents the transmission behavior of the measurement arrangement,
    wherein the data processing device (40) is configured to determine the value indicative of the impedance of the suspension according to the following formula:

$$Z = k\,\frac{1}{(\lambda_1 - \lambda_2)}\left(G_{el}^{-1}\left(Z_{sig}\right)\Big|_1 - G_{el}^{-1}\left(Z_{sig}\right)\Big|_2\right),$$

    wherein $Z_{sig}|_1$ denotes the first impedance measurement value, $Z_{sig}|_2$ denotes the second impedance measurement value, $G_{el}^{-1}$ denotes the correction function representing the transmission behavior of the measurement arrangement, $\lambda_1$ denotes a first geometry factor representing the measurement geometry of the first pair of the at least three measurement electrodes, $\lambda_2$ denotes a second geometry factor representing the measurement geometry of the second pair of the at least three measurement electrodes, and k denotes a proportionality constant.

17. A computer program comprising program instructions which, when executed on a data processing system, perform a method according to any of claims 1 to 11, wherein the individual steps of the method are initiated by the program instructions and executed by components of a sensor or are executed in the data processing system itself.

**Revendications**

1. Procédé pour déterminer une valeur indiquant l'impédance d'une suspension dans le cadre d'une spectroscopie d'impédance, comprenant les étapes suivantes :

generer un courant d'excitation oscillant avec une fréquence d'excitation à travers la suspension, le courant d'excitation étant généré à travers la suspension au moyen d'une paire d'électrodes d'excitation (8, 10), déterminer une première valeur de mesure d'impédance sur la base du courant d'excitation et d'une première tension à une première paire d'électrodes de mesure (11, 12), déterminer une deuxième valeur de mesure d'impédance sur la base du courant d'excitation et d'une deuxième tension à une deuxième paire d'électrodes de mesure (11, 13; 13, 14), la première paire d'électrodes de mesure et la deuxième paire d'électrodes de mesure étant des paires d'électrodes de mesure différentes, déterminer la valeur indiquant l'impédance de la suspension en mettant en relation la première valeur de mesure d'impédance et la deuxième valeur de mesure d'impédance, où la détermination de la valeur indiquant l'impédance de la suspension comprend la formation d'une différence entre une première valeur d'impédance ajustée et une deuxième valeur d'impédance ajustée, la première valeur d'impédance ajustée et la deuxième valeur d'impédance ajustée étant obtenues par application d'une fonction de correction sur la première valeur de mesure d'impédance et la deuxième valeur de mesure d'impédance, la fonction de correction représentant le comportement de transmission du dispositif de mesure, où la mise en relation de la première valeur de mesure d'impédance et de la deuxième valeur de mesure d'impédance s'effectue selon la formule suivante :

$$Z = k \, \frac{1}{(\lambda_1 - \lambda_2)} \left( G_{el}^{-1}(Z_{sig})\big|_1 - G_{el}^{-1}(Z_{sig})\big|_2 \right)$$

,

où $Z_{sig}|_1$ désigne la première valeur de mesure d'impédance, $Z_{sig}|_2$ désigne la deuxième valeur de mesure d'impédance, $G_{el}^{-1}$ désigne la fonction de correction représentant le comportement de transmission du dispositif de mesure, $\lambda_1$ désigne un premier facteur de géométrie représentant la géométrie de mesure de la première paire d'électrodes de mesure, $\lambda_2$ désigne un deuxième facteur de géométrie représentant la géométrie de mesure de la deuxième paire d'électrodes de mesure, et k désigne une constante de proportionnalité.

2. Procédé selon la revendication 1,

où la première paire d'électrodes de mesure est composée d'une première électrode de mesure (11) et d'une deuxième électrode de mesure (12) et où la deuxième paire d'électrodes de mesure est composée de la première électrode de mesure (11) et d'une troisième électrode de mesure (13), ou où la première paire d'électrodes de mesure est composée d'une première électrode de mesure (11) et d'une deuxième électrode de mesure (12) et où la deuxième paire d'électrodes de mesure est composée d'une troisième électrode de mesure (13) et d'une quatrième électrode de mesure (14).

3. Procédé selon l'une des revendications précédentes, comprenant en outre :

mesurer la première tension à la première paire d'électrodes de mesure, et mesurer la deuxième tension à la deuxième paire d'électrodes de mesure, où la mesure de la première tension et la mesure de la deuxième tension s'effectuent essentiellement simultanément; ou comprenant en outre:

mesurer la première tension à la première paire d'électrodes de mesure,

et

mesurer la deuxième tension à la deuxième paire d'électrodes de mesure,

où la mesure de la première tension et la mesure de la deuxième tension s'effectuent de manière décalée dans le temps.

4. Procédé selon l'un des revendications précédentes, où la détermination de la première valeur de mesure d'impédance et la détermination de la deuxième valeur de mesure d'impédance comprend :

échantillonnage du courant d'excitation,

échantillonnage de la première tension, et

échantillonnage de la deuxième tension ;

où le procédé comprend en particulier les étapes suivantes :

régler un premier taux d'échantillonnage pour l'échantillonnage du courant d'excitation,

régler un deuxième taux d'échantillonnage pour l'échantillonnage de la première tension, et

régler un troisième taux d'échantillonnage pour l'échantillonnage de la deuxième tension,

où le premier taux d'échantillonnage, le deuxième taux d'échantillonnage et le troisième taux d'échantillonnage sont réglés au moins à 4 fois la fréquence d'excitation du courant d'excitation, en particulier essentiellement à 4 fois la fréquence d'excitation du courant d'excitation.

5. Procédé selon la revendication 4, où l'étape de détermination de la première valeur de mesure d'impédance comprend la réalisation d'une première transformation de Fourier complexe sur la base des valeurs échantillonnées du courant d'excitation et des valeurs échantillonnées de la première tension, et où l'étape de détermination de la deuxième valeur de mesure d'impédance comprend la réalisation d'une deuxième transformation de Fourier complexe sur la base des valeurs échantillonnées du courant d'excitation et des valeurs échantillonnées de la deuxième tension.

6. Procédé selon l'une des revendications précédentes, comprenant en outre:

déterminer une troisième valeur de mesure d'impédance sur la base du courant d'excitation et d'une troisième tension à une troisième paire d'électrodes de mesure, et

déterminer la valeur indiquant l'impédance de la suspension en mettant en relation la première valeur de mesure d'impédance, la deuxième valeur de mesure d'impédance et la troisième valeur de mesure d'impédance.

où la détermination de la valeur indiquant l'impédance de la suspension comprend une première formation de différence entre la première valeur d'impédance ajustée et la deuxième valeur d'impédance ajustée, une deuxième formation de différence entre la première valeur d'impédance ajustée et une troisième valeur d'impédance ajustée, et une troisième formation de différence entre la deuxième valeur d'impédance ajustée et la troisième valeur d'impédance ajustée, la première valeur d'impédance ajustée, la deuxième valeur d'impédance ajustée et la troisième valeur d'impédance ajustée étant obtenues par application de la fonction de correction sur la première valeur de mesure d'impédance, la deuxième valeur de mesure d'impédance et la troisième valeur de mesure d'impédance.

7. Procédé selon la revendication 6, où la détermination de la valeur indiquant l'impédance de la suspension comprend une première formation de différence entre le premier facteur de géométrie et le deuxième facteur de géométrie, une deuxième formation de différence entre le premier facteur de géométrie et un troisième facteur de géométrie, et une troisième formation de différence entre le deuxième facteur de géométrie et le troisième facteur de géométrie, le troisième facteur de géométrie représentant la géométrie de mesure de la troisième paire d'électrodes de mesure;

où la détermination de la valeur indiquant l'impédance de la suspension s'effectue en particulier selon la formule suivante :

$$Z^2 = k_2 \frac{\lambda_3 \left( G_{el}^{-1}(Z_{sig})|_2 - G_{el}^{-1}(Z_{sig})|_1 \right)}{(\lambda_1 - \lambda_2)(\lambda_1 - \lambda_3)(\lambda_2 - \lambda_3)} +$$

$$+ k_2 \frac{\lambda_2 \left( G_{el}^{-1}(Z_{sig})|_1 - G_{el}^{-1}(Z_{sig})|_3 \right)}{(\lambda_1 - \lambda_2)(\lambda_1 - \lambda_3)(\lambda_2 - \lambda_3)} +$$

$$+ k_2 \frac{\lambda_1 \left( G_{el}^{-1}(Z_{sig})|_3 - G_{el}^{-1}(Z_{sig})|_2 \right)}{(\lambda_1 - \lambda_2)(\lambda_1 - \lambda_3)(\lambda_2 - \lambda_3)}$$

,

où $Z_{sig|3}$ désigne la troisième valeur de mesure d'impédance, $\lambda_3$ désigne un troisième facteur de géométrie représentant la géométrie de mesure de la troisième paire d'électrodes de mesure, et $k_2$ désigne une constante de proportionnalité.

8. Procédé de détermination d'une valeur indiquant l'impédance d'une suspension dans le cadre d'une spectroscopie d'impédance, comprenant les étapes suivantes :

générer une tension d'excitation oscillant à une fréquence d'excitation appliquée à la suspension, la tension d'excitation étant générée par la suspension au moyen d'une paire d'électrodes d'excitation (8, 10), déterminer une première valeur de mesure d'impédance basée sur la tension d'excitation et un premier courant traversant une première paire d'électrodes de mesure, déterminer une deuxième valeur de mesure d'impédance basée sur la tension d'excitation et un deuxième courant traversant une deuxième paire d'électrodes de mesure, la première paire d'électrodes de mesure et la deuxième paire d'électrodes de mesure étant des paires d'électrodes de mesure différentes, déterminer la valeur indiquant l'impédance de la suspension en mettant en relation la première valeur de mesure d'impédance et la deuxième valeur de mesure d'impédance, la détermination de la valeur indiquant l'impédance de la suspension comprend la formation de la différence entre une première valeur d'impédance ajustée et une deuxième valeur d'impédance ajustée, la première valeur d'impédance ajustée et la deuxième valeur d'impédance ajustée étant obtenues par application d'une fonction de correction à la première valeur de mesure d'impédance et à la deuxième valeur de mesure d'impédance, la fonction de correction représentant de préférence le comportement de transfert de l'agencement de mesure, où la mise en relation de la première valeur de mesure d'impédance et de la deuxième valeur de mesure d'impédance se fait selon la formule suivante :

$$Z = k \frac{1}{(\lambda_1 - \lambda_2)} \left( G_{el}^{-1}(Z_{sig})|_1 - G_{el}^{-1}(Z_{sig})|_2 \right)$$

,

où $Z_{sig|1}$ désigne la première valeur de mesure d'impédance, $Z_{sig|2}$ désigne la deuxième valeur de mesure d'impédance, $G_{el}^{-1}$ désigne la fonction de correction représentant le comportement de transfert de l'agencement de mesure, $\lambda_1$ désigne un premier facteur géométrique représentant la géométrie de mesure de la première paire d'électrodes de mesure, $\lambda_2$ désigne un deuxième facteur géométrique représentant la géométrie de mesure de la deuxième paire d'électrodes de mesure, et k désigne une constante de proportionnalité.

9. Procédé pour dériver au moins une propriété caractéristique d'une suspension, comprenant les étapes suivantes :

réaliser plusieurs fois le procédé pour déterminer une valeur indiquant l'impédance d'une suspension selon l'une des revendications précédentes en utilisant une pluralité de fréquences d'excitation différentes et déterminer une pluralité de valeurs indiquant l'impédance de la suspension pour la pluralité de fréquences d'excitation différentes, dériver une pluralité de valeurs indiquant la permittivité de la suspension sur la base de la pluralité de valeurs indiquant l'impédance de la suspension, et

dériver la ou les propriétés caractéristiques de la suspension en mettant en relation la pluralité de valeurs indiquant la permittivité de la suspension.

10. Procédé selon la revendication 9,

où ledit procédé pour déterminer une valeur indiquant l'impédance d'une suspension est réalisé pour entre 2 et 50 fréquences d'excitation différentes, en particulier pour entre 10 et 40 fréquences d'excitation différentes, plus particulièrement pour entre 20 et 30 fréquences d'excitation différentes ;
et/ou
où les fréquences d'excitation différentes proviennent d'une plage de fréquences de 100 kHz à 10 MHz, en particulier d'une plage de fréquences de 50 kHz à 20 MHz.

11. Procédé selon la revendication 9 ou 10,
où la dérivation de la ou des propriétés caractéristiques de la suspension comprend la création d'un profil des valeurs indiquant la permittivité de la suspension sur les différentes fréquences d'excitation ;
et/ou
où la suspension est une population cellulaire et où la ou les propriétés caractéristiques de la suspension comprennent au moins une propriété parmi le nombre de cellules vivantes, la taille des cellules et l'homogénéité des cellules.

12. Capteur (2) pour déterminer une valeur indiquant l'impédance d'une suspension, comprenant :

une circuit oscillateur (16),
une paire d'électrodes d'excitation (8, 10) couplée au circuit oscillateur, où un courant d'excitation oscillant avec une fréquence d'excitation peut être généré à travers la suspension au moyen de la paire d'électrodes d'excitation,
au moins trois électrodes de mesure pour mesurer une première tension dans la suspension entre une première paire des au moins trois électrodes de mesure et une deuxième tension dans la suspension entre une deuxième paire des au moins trois électrodes de mesure, la première paire d'électrodes de mesure et la deuxième paire d'électrodes de mesure étant des paires d'électrodes de mesure différentes, et
un dispositif de traitement de données (40) configuré pour déterminer une première valeur de mesure d'impédance sur la base du courant d'excitation et de la première tension, déterminer une deuxième valeur de mesure d'impédance sur la base du courant d'excitation et de la deuxième tension, et déterminer la valeur indiquant l'impédance de la suspension en mettant en relation la première valeur de mesure d'impédance et la deuxième valeur de mesure d'impédance;
où le dispositif de traitement de données (40) est configuré pour déterminer la valeur indiquant l'impédance de la suspension par formation d'une différence entre une première valeur d'impédance ajustée et une deuxième valeur d'impédance ajustée, où le dispositif de traitement de données est configuré pour obtenir la première valeur d'impédance ajustée et la deuxième valeur d'impédance ajustée par application d'une fonction de correction sur la première valeur de mesure d'impédance et la deuxième valeur de mesure d'impédance, la fonction de correction représentant le comportement de transmission du dispositif de mesure,
où le dispositif de traitement de données (40) est configuré pour déterminer la valeur indiquant l'impédance de la suspension selon la formule suivante :

$$Z = k \frac{1}{(\lambda_1 - \lambda_2)} \left( G_{el}^{-1}(Z_{sig})\big|_1 - G_{el}^{-1}(Z_{sig})\big|_2 \right),$$

où $Z_{sig|1}$ désigne la première valeur de mesure d'impédance, $Z_{sig|2}$ désigne la deuxième valeur de mesure d'impédance, $G_{el}^{-1}$ désigne la fonction de correction représentant le comportement de transmission du dispositif de mesure, $\lambda_1$ désigne un premier facteur de géométrie représentant la géométrie de mesure de la première paire d'électrodes de mesure, $\lambda_2$ désigne un deuxième facteur de géométrie représentant la géométrie de mesure de la deuxième paire d'électrodes de mesure, et k désigne une constante de proportionnalité.

13. Capteur (2) selon la revendication 12, où les au moins trois électrodes de mesure sont disposées entre la paire d'électrodes d'excitation (8, 10).

**14.** Capteur (2) selon les revendications 12 ou 13,

où les au moins trois électrodes de mesure sont au moins quatre électrodes de mesure, où la première paire des au moins quatre électrodes de mesure est composée d'une première électrode de mesure (11) et d'une deuxième électrode de mesure (12) et où la deuxième paire des au moins quatre électrodes de mesure est composée d'une troisième électrode de mesure (13) et d'une quatrième électrode de mesure (14);
où la troisième et la quatrième électrode de mesure (13, 14) sont en particulier disposées entre la première et la deuxième électrode de mesure (11, 12) et/ou où la troisième et la quatrième électrode de mesure (13, 14) sont en particulier disposées sur un autre côté du capteur que la première et la deuxième électrode de mesure (11, 12).

**15.** Capteur (2) selon l'une des revendications 12 à 14, où le circuit oscillateur (16) est couplé à la paire d'électrodes d'excitation (8, 10) par un transformateur (22), où le transformateur (22) a en particulier une capacité parallèle de 0,5 à 10 pF, plus particulièrement de 1 à 5 pF.

**16.** Capteur (2) pour déterminer une valeur indiquant l'impédance d'une suspension, comprenant :

un circuit oscillateur (16),
une paire d'électrodes d'excitation (8, 10), qui sont couplées au circuit oscillateur, par lequel une tension d'excitation oscillant à une fréquence d'excitation et appliquée à la suspension peut être générée via la paire d'électrodes d'excitation
au moins trois électrodes de mesure pour mesurer un premier courant dans la suspension entre une première paire des au moins trois électrodes de mesure et un deuxième courant dans la suspension entre une deuxième paire des au moins trois électrodes de mesure, où la première paire d'électrodes de mesure et la deuxième paire d'électrodes de mesure sont des paires d'électrodes de mesure différentes, et
un dispositif de traitement de données (40) configuré pour déterminer une première valeur de mesure d'impédance sur la base de la tension d'excitation et du premier courant, déterminer une deuxième valeur de mesure d'impédance sur la base de la tension d'excitation et du deuxième courant, et déterminer la valeur indiquant l'impédance de la suspension en mettant en relation la première valeur de mesure d'impédance et la deuxième valeur de mesure d'impédance;
où le dispositif de traitement de données (40) est configuré pour déterminer la valeur indiquant l'impédance de la suspension par formation d'une différence entre une première valeur d'impédance ajustée et une deuxième valeur d'impédance ajustée, où le dispositif de traitement de données est configuré pour obtenir la première valeur d'impédance ajustée et la deuxième valeur d'impédance ajustée par application d'une fonction de correction sur la première valeur de mesure d'impédance et la deuxième valeur de mesure d'impédance, la fonction de correction représentant le comportement de transmission du dispositif de mesure,
où le dispositif de traitement de données (40) est configuré pour déterminer la valeur indiquant l'impédance de la suspension selon la formule suivante :

$$Z = k \frac{1}{\left|\lambda_1 - \lambda_2\right|}\left( G_{el}^{-1}\left(Z_{sig}\right)\big|_1 - G_{el}^{-1}\left(Z_{sig}\right)\big|_2 \right),$$

où $Z_{sig|1}$ désigne la première valeur de mesure d'impédance, $Z_{sig|2}$ désigne la deuxième valeur de mesure d'impédance, $G_{el}^{-1}$ désigne la fonction de correction représentant le comportement de transmission du dispositif de mesure, $\lambda_1$ désigne un premier facteur de géométrie représentant la géométrie de mesure de la première paire d'électrodes de mesure, $\lambda_2$ désigne un deuxième facteur de géométrie représentant la géométrie de mesure de la deuxième paire d'électrodes de mesure, et k désigne une constante de proportionnalité.

**17.** Programme informatique contenant des instructions de programme qui, lorsqu'elles sont exécutées sur un dispositif de traitement de données, réalisent un procédé selon l'une des revendications 1 à 11, les différentes étapes du procédé étant provoquées par les instructions de programme et exécutées par des composants d'un capteur ou exécutées directement dans le dispositif de traitement de données.

# Fig. 1

**Fig. 2**

EP 3 887 810 B1

**Fig. 3**

# Fig. 4

# Fig. 5

Fig. 6

Fig. 7

EP 3 887 810 B1

# Fig. 8

Fig. 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- Cell culture monitoring by impedance mapping using a multielectrode scanning impedance spectroscopy system (CellMap). **RAHMAN A R A et al.** Physiological Measurement. Institute of physics publishing, 01 June 2008, vol. 29